# EUROPEAN PATENT SPECIFICATION

(11) **EP 4 531 856 B1**
(45) Date of publication and mention of the grant of the patent: **22.04.2026**
(21) Application number: 23730757.4
(22) Date of filing: 01.06.2023
(51) Int. Cl.: A61K 31/4985, A61K 31/137, A61K 31/519, A61K 45/06, A61P 37/08

(54) **BRUTON'S KINASE INHIBITORS FOR THE TREATMENT OF A SUDDEN ALLERGIC REACTION**
BRUTON-KINASE-INHIBITOREN ZUR BEHANDLUNG EINER PLÖTZLICH AUFTRETENDEN ALLERGISCHEN REAKTION
INHIBITEURS DE LA KINASE DE BRUTON POUR LE TRAITEMENT D'UNE RÉACTION ALLERGIQUE SOUDAINE

(30) Priority: 01.06.2022 EP 22176862
(43) Date of publication of application: 09.04.2025
(73) Proprietor: ALK-Abelló A/S, 2970 Hørsholm (DK)
(72) Inventor: JACOBI, Hugo, Henrik, 2970 Hørsholm (DK); LUND, Gitte, 2970 Hørsholm (DK)
(74) Representative: Inspicos P/S
(86) International application number: PCT/EP2023/064721
(87) International publication number: WO 2023/232958

(56) References cited:
- WO-A1-2022/012550
- US-A1- 2017 044 166
- NADEEM AHMED ET AL: "Inhibition of Bruton's tyrosine kinase and IL-2 inducible T-cell kinase suppresses both neutrophilic and eosinophilic airway inflammation in a cockroach allergen extract-induced mixed granulocytic mouse model of asthma using preventative and therapeutic strategy", PHARMACOLOGICAL RESEARCH, ELSEVIER, AMSTERDAM, NL, vol. 148, 7 September 2019 (2019-09-07), XP085875208, ISSN: 1043-6618, [retrieved on 20190907], DOI: 10.1016/J.PHRS.2019.104441
- DISPENZA MELANIE C ET AL: "Bruton's tyrosine kinase inhibition effectively protects against human IgE-mediated anaphylaxis", 2 June 2020 (2020-06-02), XP055980743, Retrieved from the Internet <URL:https://www.ncbi.nlm.nih.gov/pmc/articles/PMC7456252/pdf/jci-130-138448.pdf> [retrieved on 20221114]
- MORRIS SUZANNE C ET AL: "Optimizing drug inhibition of IgE-mediated anaphylaxis in mice", JOURNAL OF ALLERGY AND CLINICAL IMMUNOLOGY, ELSEVIER, AMSTERDAM, NL, vol. 149, no. 2, 26 June 2021 (2021-06-26), pages 671, XP086949227, ISSN: 0091-6749, [retrieved on 20210626], DOI: 10.1016/J.JACI.2021.06.022
- REGAN JENNIFER A ET AL: "Ibrutinib, a Bruton's tyrosine kinase inhibitor used for treatment of lymphoproliferative disorders, eliminates both aeroallergen skin test and basophil activation test reactivity", JOURNAL OF ALLERGY AND CLINICAL IMMUNOLOGY, ELSEVIER, AMSTERDAM, NL, vol. 140, no. 3, 4 April 2017 (2017-04-04), pages 875, XP085165327, ISSN: 0091-6749, DOI: 10.1016/J.JACI.2017.03.013

## Description

### FIELD OF THE INVENTION

The present invention relates to a method for treating suddenly occurring allergic reactions, such as acute allergic reactions, in a human through administration of a Bruton's tyrosine kinase inhibitor (BTKi). In particular, the present invention relates to a method for emergency treatment of a human who has clinical signs or symptoms of a severe allergic reaction, such as anaphylaxis, or expect to get a severe allergic reaction, such as anaphylaxis, due to an accidental exposure to an allergen.

### BACKGROUND OF THE INVENTION

While several treatment options exist for treating or preventing mild to moderate allergic conditions, including antihistamines, steroids and immunotherapy, there are only limited treatment options for providing a rapid relief of severe allergic reactions, and not at least when it comes to severe allergic reactions that develop rapidly following exposure to an agent able to trigger degranulation of mast cells and/or basophils, such as an allergen.

Such acute allergic reactions are potentially life-threatening and often triggered by exposure to allergens present in foods (typically foods like nuts, milk, fish, shellfish, eggs, and some fruits), medications, plants, venoms (typically wasp or bee stings). Although allergic patients intentionally seek to avoid such allergens, they may nevertheless be accidentally exposed to such allergens via stinging insects or allergens hidden in food products. Therefore, patients with a history of acute allergic reactions should be able to react fast, either following the first signs of symptoms or upon exposure to an allergen. If the treatment is not initiated immediately or soon after exposure to the allergen, the initial signs of an allergic reaction can progress to anaphylaxis that involves constriction of the airways, swelling of the throat, rapid heart rate, severe hypotension and other respiratory and cardiac symptoms that can develop and potentially present a life-threatening emergency.

The first signs of an anaphylactic reaction may look like typical allergy symptoms: a runny nose or a skin rash, but within about 15 to 60 minutes, more serious signs appear. The patient usually exhibits more than one of these signs: hypotension; constriction of the airways; swollen tongue or throat, which can cause wheezing and trouble breathing; weak and rapid pulse; nausea, vomiting or diarrhoea; dizziness or fainting.

Today, the only available treatment option with life-saving effect for treating such acute allergic reactions, including anaphylactic reactions, is with epinephrine. Epinephrine may be self-administered by the patient using an autoinjector for subcutaneous injection or it may be administered intravenously at the emergency room. Epinephrine restores blood pressure and counteracts the detrimental signs of anaphylactic reactions by stimulating alpha- and beta-adrenergic receptors. This leads to vasoconstriction in the precapillary arterioles of the skin, mucosa and kidneys, and smoothed muscle contraction in the venous vascular bed, which results in increased peripheral vascular resistance and blood pressure. Subsequently, tissue edema is reduced. Stimulation of the beta receptors increases the heart rate and contractility of the cardiac muscle.

Despite that epinephrine treatment is the cornerstone of anaphylaxis management and the first-line treatment in emergency situations, the epinephrine treatment does not treat the underlying immunological response, e.g., reduces mast cell and/or basophil degranulation, which triggers the life-threatening acute allergic reaction including anaphylaxis. A patient is advised to administer epinephrine immediately at the first sign of a serious allergic reaction, or anaphylaxis. Since the progression of an acute allergic reaction can vary from minutes to hours following allergen exposure, it causes uncertainty for the patient as to when to initiate emergency treatment with epinephrine after realizing that they have been exposed to an allergen, which potentially could result in a life-threatening allergic condition. For people with allergy, this can cause anxiety with a compromised health-related quality of life. In addition, many patients in need of epinephrine emergency treatment are delayed in administering the epinephrine, because they have needle phobia, or have difficulties in managing the epinephrine autoinjector. This may increase the risk of serious complications, need of hospitalisation and fatal outcomes.

Allergic reactions, whether leading to a mild or severe allergic reaction, including anaphylaxis, may be mechanistically triggered by the activation of pre-sensitised basophils and mast cells by allergens that cross-link allergen-specific IgE-antibodies bound to the high affinity IgE-receptor (designated FcεRI) present on the surface of basophils and mast cells. Upon activation of the cells, a cascade of downstream reactions is immediately initiated inside the cells finally causing the burst of the cells to release and initiate de-novo synthesis of several allergic mediators (including histamine, tryptase, prostaglandins, leukotrienes, and cytokines), which result in rapid induction of allergic symptoms with peak severity within minutes to hours after allergen exposure. Thus, in the pathogenesis of the allergic response including anaphylaxis the common upstream event is the exposure of mast cells / basophils to an agent able to trigger mast cell / basophil degranulation (for example by allergens cross-linking IgE preloaded on FcεRI) thereby triggering the cellular degranulation with the release of allergic mediators, which then results in the immediate or delayed occurrence of clinical signs or symptoms of allergy including anaphylaxis.

To alleviate the allergic reaction in association with anaphylaxis, epinephrine treatment is often followed by second-line treatment with intravenous administration of antihistamines and/or glucocorticosteroids, which typically takes place at an emergency room or hospital and not as self-medication.

There is an unmet need for improved management of acute allergic reactions, including anaphylaxis, with medicaments able to rapidly attenuate the underlying release of allergic mediators during an ongoing allergic episode to quickly dampen the ongoing allergic reaction and thus preventing or postponing the need of epinephrine treatment and/or second line treatment. Preferably, the medicaments might be able to have a fast onset of action which may allow for the medicament being administered as a single dose shortly after allergen exposure or before the occurrence of the first signs of symptoms of an allergic reaction which could progress into anaphylaxis.

Bruton's tyrosine kinase (BTK) is a kinase known to play a role in different human immune cells. In B-cells, BTK is activated upon antigen binding to the B-cell receptor (BCR). In antigen dependent BCR signaling, BTK can be activated by PI3K or SYK. The primary substrate for BTK is PLC-gamma-2 leading to activation of NFAT, NFkB and MYC, which are all key components in regulation of the cellular function, including proliferation, survival, and differentiation (Wen et al 2021).

In both mast cells and basophils, BTK is activated downstream the IgE high affinity receptor, FcεRI when allergens are cross-linking IgE antibodies bound to FcεRI. BTK activation culminates in further downstream activation of multiple pathways, including MAP kinases like P38, ERK and JNK, as well as activation of NF-kB and NFAT. These pathways play a key role in activation of mast cells and their degranulation and production/release of allergic mediators such as histamine, pro-inflammatory cytokines, and various enzymes (Ellmeier et al. 2011).

BTKis are known to be associated with adverse effects that develop following an extended period of use (Lipsky and Lamanna 2020) and are therefore mainly used for the treatment of malign diseases. Today, there are several BTKis approved for the treatment of B-cell malignancies, such as non-Hodgkin lymphomas and chronic lymphocytic leukemia. Ibrutinib was the first BTKi approved by the FDA in 2013 and second generations BTKis acalabrutinib and zanubrutinib with fewer off target adverse events were approved in 2017 and 2019, respectively (Wen et al 2021). The BTKis are administered perorally, such as by tablets, capsules or suspensions.

Despite that there are numerous BTKis in development, none have been exploited in larger clinical trials for the treatment of allergy.

However, pretreatment of basophil cells with BTKis (including Ibrutinib) before allergen exposure has been shown to inhibit IgE-mediated histamine release and this basophil-targeting effect of ibrutinib was confirmed by demonstrating that IgE-dependent histamine release in ex-vivo blood basophils was largely suppressed in a leukemia patient treated with ibrutinib (Smiljkovic et al., 2017). Further evidence on BTKis having a role in relation to allergy has been published by Dispenza et al. (2020) and Dispenza et al. (2018). According to Dispenza et al. (2018), peanut- and/or tree nut-allergic subjects were administering ibrutinib daily for up to seven days and the treatment effect was evaluated by skin prick testing (SPTs) and ex-vivo basophil activation testing (BAT) before, during, and after ibrutinib treatment. It was found that short-term ibrutinib therapy before allergen exposure can reduce mast cell and basophil reactivity to food. In an anaphylactic mouse model, it was demonstrated that pretreatment with just two oral doses of the BTKi acalabrutinib 16 hours and 4 hours before allergen exposure completely prevented moderate IgE-mediated anaphylaxis in these mice and significantly protected against death during severe anaphylaxis (Dispenza et al. 2020). Further, it was found that BTKi pretreatment of skin-derived mast cells (SDMCs) 15 minutes before allergen exposure could prevent IgE-mediated activation of the SDMCs. Accordingly, it has been found that BTKis are useful as prophylactic treatment before allergen exposure and Dispenza MC (2021) suggests the use of BTKi for prophylactic treatment of IgE-mediated food allergies or as an adjuvant therapy to food oral immunotherapy (OIT). Notably, the prophylactic treatment seems mostly relevant if the patient is expecting a near-future planned exposure to an allergen and will not be useful in situation of accidental exposure.

All FDA approved BTKis are for oral administration, despite that the bioavailability is rather low. It has been reported that the BTKi, PRN473, in development by Principia Biopharma is for topical administration and may have the potential to be used in the treatment of allergic rhinitis and conjunctivitis if it has adequate penetration of mucosal surfaces (Dispenza MC, 2021).

NADEEM AHMED ET AL: "Inhibition of Bruton's tyrosine kinase and IL-2 inducible T-cell kinase suppresses both neutrophilic and eosinophilic airway inflammation in a cockroach allergen extract-induced mixed granulocytic mouse model of asthma using preventative and therapeutic strategy", PHARMACOLOGICAL RESEARCH, ELSEVIER, AMSTERDAM, NL, vol. 148, 7 September 2019, discloses the use of the BTK inhibitor ibrutinib to reduce mixed granulocytic airway inflammation in a mouse model of asthma, wherein the drug is administered intranasally before each allergen challenge in both preventive and so-called therapeutic regimens.

### OBJECT OF THE INVENTION

The object of the invention is to provide treatment options able to disrupt the allergic mediator release of an ongoing allergic response, in particular a response occurring just after mast cell activation caused by an episodic exposure to a triggering allergen. Advantageously, the treatment can be initiated just after the allergic subject is being exposed to an allergen expected to or triggering mast cell degranulation, which may result in an allergic reaction with severe modality, including anaphylaxis. Alternatively, the BTKi may be administered before or just after the allergic subject is experiencing the first signs or symptoms of an allergic reaction triggered by mast cell degranulation.

### SUMMARY OF THE INVENTION

The invention is defined by the appended claims. Any subject-matter falling outside the scope of the claims is provided for information purposes, only. The references to methods of treatment in this description are to be interpreted as references to the compounds, pharmaceutical compositions and medicaments of the present invention for use in a method for treatment of the human (or animal) body by therapy.

The present inventors have surprisingly found that a single dose administration of a BTKi can reduce an ongoing allergic reaction in that BTKi administered after the mast cells / basophils have been triggered by allergen exposure has been shown to be effective in reducing the mast cell and/or basophil degranulation. Advantageously, mast cell and/or basophil degranulation can be reduced, prevented, or inhibited without the need of administering several doses of a BTKi for a period up to the subject's exposure to an agent triggering said degranulation. Extended periods with BTKi administering may cause adverse events that are not acceptable for patients suffering from allergy.

As shown in example 1, the addition of a BTKi to already activated basophil cells was able to attenuate the level of allergic mediator release significantly within a short time. Thus, it is envisaged that BTKis have very fast onset of action and would be able to attenuate an acute allergic reaction to effectively reduce or prevent the progression into a severe allergic reaction, including anaphylaxis, which might require epinephrine treatment.

Further shown in example 2, BTKi was able to reduce anaphylaxis in two different mouse models, both when the BTKi was administered before induction of anaphylaxis (reference example), but also, and
most important, when the BTKi was administered to the mice after anaphylaxis was induced. Thus, showing that the BTKi can work in an *in vivo* system with high complexity.

The present inventors therefore suggest administering a single dose of BTKi at, or soon after the exposure to an agent able to trigger the degranulation (e.g., an allergen) in order to alleviate an allergic reaction developing as a consequence of the degranulation of mast cells and/or basophils. Further, this may consequently decrease the risk of the allergic reaction progressing into a severe and life-threatening allergic reaction. Depending on the pharmacological profile of the BTKi and severity of the allergic reaction, a repeat dose of the BTKi might be administered within 24 hours from the first dose.

Thus, the present invention deals with a BTKi as defined in claim 1 for use in a method of treating an allergic reaction in a subject in need thereof, wherein the method comprises administering a therapeutically effective dose of a Bruton's tyrosine kinase inhibitor (BTKi) to said subject, wherein the BTKi is administered within a period ranging from the subject's exposure to an agent triggering said allergic reaction until 24 hours after said exposure.

Thus, a first aspect of the present disclosures relates to a method for reducing degranulation of mast cells and/or basophils in a subject in need thereof, which degranulation is triggered by exposure to an agent able to activate mast cells and/or basophils, the method comprising administering a therapeutically effective dose of a Bruton's tyrosine kinase inhibitor (BTKi) to said subject, wherein the dose of the BTKi is administered within a period selected from any one of i) close to said exposure, ii) close to the initiation of mast cell and/or basophil degranulation and/or iii) prior to, after or when the allergic subject has experienced the first sign or symptoms of the allergic reaction caused by exposure to said agent able to trigger the degranulation (e.g. an allergen).

Such methods can be applied for many various purposes for the benefit of subjects in need thereof, such as subjects experiencing an episode of an agent able to trigger the degranulation and who are in risk of developing a severe allergic reaction due to this episode of exposure. The following purposes shall be a non-limiting list of purposes:
A BTKi may be administered for many various purposes, such as for i) alleviating an allergic reaction, such as an acute allergic reaction, in a subject in need thereof, the allergic reaction might optionally be a severe allergic reaction or anaphylaxis; ii) preventing an ongoing allergic reaction and/or ongoing degranulation of mast cells and/or basophils to progress into a severe allergic reaction or anaphylaxis; iii) mitigating the risk of developing anaphylaxis; iv) mitigation the risk of developing a delayed allergic reaction or anaphylaxis, such as biphasic anaphylaxis; v) emergency treatment of a subject who has been accidentally exposed to an agent causing or triggering mast cell and/or basophil degranulation; vi) preventing, postponing or reducing the need for epinephrine administration and/or second line treatment with antihistamine and/or corticosteroids.

As mast cell and/or basophil degranulation is a key event in allergic reactions and anaphylaxis, the purpose of the method of reducing degranulation of mast cells and/or basophils might include the alleviation or treatment of an ongoing allergic reaction with the outcome that anaphylaxis is prevented or postponed, epinephrine treatment or second-line treatment will not be required or ultimately that hospitalisation might not be required.

Therefore, a second aspect relates to a method for treatment or alleviation of an allergic reaction in a subject in need thereof (e.g., an allergic subject), the method comprising administering a therapeutically effective dose of a Bruton's tyrosine kinase inhibitor (BTKi) to said subject and wherein the treatment with the BTKi (i.e. the administering of the BTKi) is initiated close to said exposure and/or close to the initiation of the mast cell degranulation and/or close to the development of an allergic reaction in response to the degranulation. It might be understood that the allergic reaction is triggered by exposure to an agent able to activate mast cells and/or basophils, such as an agent able to trigger degranulation of mast cells and/or basophils (e.g., an allergen). It might further be understood that treatment with the BTKi (i.e. the administering of the BTKi) is initiated after the subject in need thereof (e.g., allergic subject) has been exposed to the agent able to trigger the degranulation (e.g., an allergen) or ii) wherein the treatment (i.e. the administering of the BTKi) is initiated before, after or when the allergic subject has experienced the first sign or symptoms of the allergic reaction caused by exposure to said agent able to trigger the degranulation (e.g., an allergen).

In other words, the second aspect relates to a method for alleviation or treating an allergic reaction, optionally a severe allergic reaction or anaphylaxis, in a subject in need thereof, comprising reducing degranulation of mast cells and/or basophils that is triggered by the subject's exposure to an allergen by administering a therapeutically effective dose of a BTKi to said subject, wherein the dose of the BTKi is administered within a period close to said exposure and/or close to the initiation of mast cell degranulation, i.e. as presently claimed.

Further aspects relate to:
A method of treating an allergic reaction, such as a type-I allergic reaction, in a subject in need thereof, e.g., an allergic subject, wherein the method comprises administering a therapeutically effective dose of a BTKi to said subject, wherein the treatment (i.e., administering of a BTKi) is initiated after the subject's exposure to an agent (such as an allergen) that is able to trigger said allergic reaction, as defined in claims.

A method for treating or alleviation of an allergic reaction in a subject in need thereof (e.g., an allergic subject), the method comprising administering a therapeutically effective dose of a BTKi to said subject and wherein the treatment with the BTKi (i.e. the administering of the BTKi) is initiated after the subject in need thereof (e.g. allergic subject) has been exposed to an agent able to trigger degranulation of mast cells and/or basophils (e.g., an allergen) or ii) wherein the treatment (i.e. the administering of the BTKi) is initiated before, after or when the allergic subject has experienced the first sign or symptoms of the allergic reaction developed in response, such as caused, by the exposure to said agent (e.g., said allergen).

A method for alleviation or treatment of an allergic reaction, optionally a severe allergic reaction or anaphylaxis, in a subject in need thereof, which allergic reaction, optionally severe allergic reaction or anaphylaxis, is developed following said subject's exposure to an agent able to trigger degranulation of mast cells and/or basophils, the method comprises reducing degranulation of mast cells and/or basophils triggered by the subject's exposure to said agent, by administering a therapeutically effective dose of a BTKi to said subject, wherein the dose of the BTKi is administered within a period close to said exposure and/or close to the initiation of mast cell degranulation, i.e. as presenty claimed.

A method for mitigating the risk of developing a delayed allergic reaction in a subject in need thereof, which delayed allergic reaction is developed following said subject's exposure to an agent able to trigger degranulation of mast cells and/or basophils, the method comprises reducing degranulation of mast cells and/or basophils triggered by the subject's exposure to said agent, by administering a therapeutically effective dose of a BTKi to said subject, wherein the dose of the BTKi is administered within a period close to said exposure and/or close to the initiation of mast cell degranulation, i.e. as presently claimed.

A method for preventing an ongoing allergic reaction and/or ongoing degranulation of mast cells and/or basophils to progress into a severe allergic reaction or anaphylaxis in a subject in need thereof, which ongoing allergic reaction and/or ongoing degranulation of mast cells and/or basophils is developed following said subject's exposure to an agent able to trigger degranulation of mast cells and/or basophils, the method comprises reducing degranulation of mast cells and/or basophils triggered by the subject's exposure to said agent by administering a therapeutically effective dose of a BTKi to said subject, wherein the dose of the BTKi is administered within a period close to said exposure and/or close to the initiation of mast cell degranulation, i.e. as presently claimed.

A method for mitigating the risk of developing anaphylaxis in a subject in need thereof, which anaphylaxis is developed following said subject's exposure to an agent able to trigger degranulation of mast cells and/or basophils, the method comprises reducing degranulation of mast cells and/or basophils triggered by the subject's exposure to said agent by administering a therapeutically effective dose of a BTKi to said subject, wherein the dose of the BTKi is administered within a period close to said exposure and/or close to the initiation of mast cell degranulation, i.e. as presently claimed.

A method for emergency treatment of a subject in need thereof, which subject has been exposed, such as accidentally exposed to an agent able to trigger mast cell and/or basophil degranulation, the method comprises reducing degranulation of mast cells and/or basophils triggered by the subject's exposure to said agent by administering a therapeutically effective dose of a BTKi to said subject, wherein the dose of the BTKi is administered within a period close to said exposure and/or close to the initiation of mast cell degranulation, i.e. as presently claimed.

A method for preventing, postponing or reducing the need for epinephrine administration and/or second-line treatment with antihistamine and/or corticosteroids in a subject in need thereof, the method comprises reducing degranulation of mast cells and/or basophils triggered by the subject's exposure to an agent able to trigger mast cells and/or basophils, by administering a therapeutically effective dose of a BTKi to said subject, wherein the dose of the BTKi is administered within a period close to said exposure and/or close to the initiation of mast cell degranulation, i.e. as presently claimed.

In all these methods, the BTKi must be administered close to the subject's exposure to an agent able to trigger the degranulation of mast cells and/or basophils (such as an allergen) and/or close to the initiation of the mast cell degranulation. This is meant to include that the BTKi is administered i) after the subject in need thereof has been exposed to the agent able to activate the mast cells/ basophils and/or ii) prior to or when the subject in need thereof has experienced the first clinical sign or symptoms of an allergic response developed in consequence of said degranulation. Therefore, a dose of BTKi is preferably administered to said subject in need thereof within a period ranging from the subject's exposure to the agent triggering said degranulation until 24 hours after said exposure, but preferably prior to or when the subject is exhibiting a clinical sign or symptom of an allergic reaction which has developed in consequence of said degranulation or exposure.

Mast cell /basophil degranulation may start immediately or soon after the subject's exposure to an agent able to trigger said degranulation in said subject, which makes it preferable to administer the BTKi soon after said exposure, such as within 30 minutes from the exposure. Alternatively formulated, all and each of the aforementioned aspects of the invention relate to a BTKi for use in the method according to any of the first, second and further aspects defined above; or the use of a BTKi for the preparation of a medicament for use in the method according to any of the first, second and further aspects defined above.

### LEGENDS TO THE FIGURE

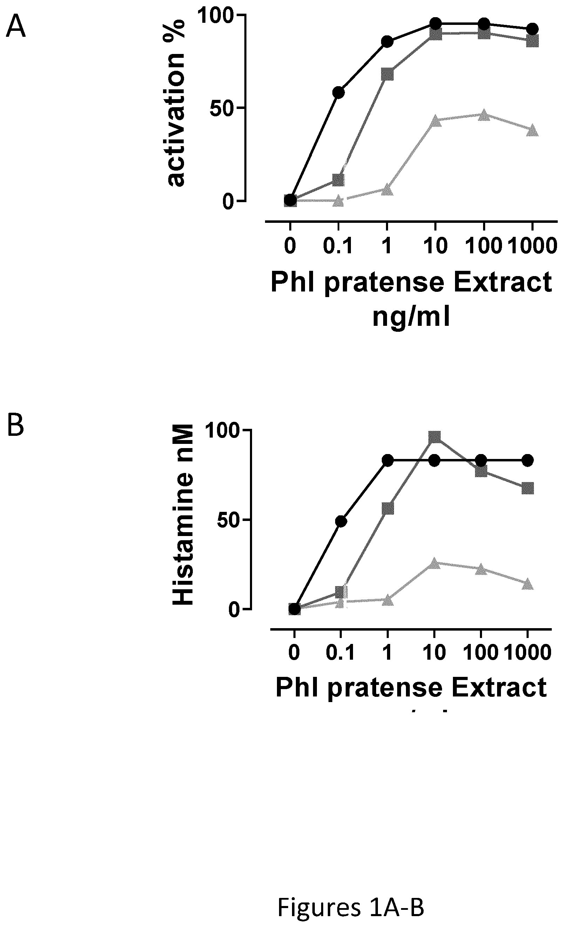

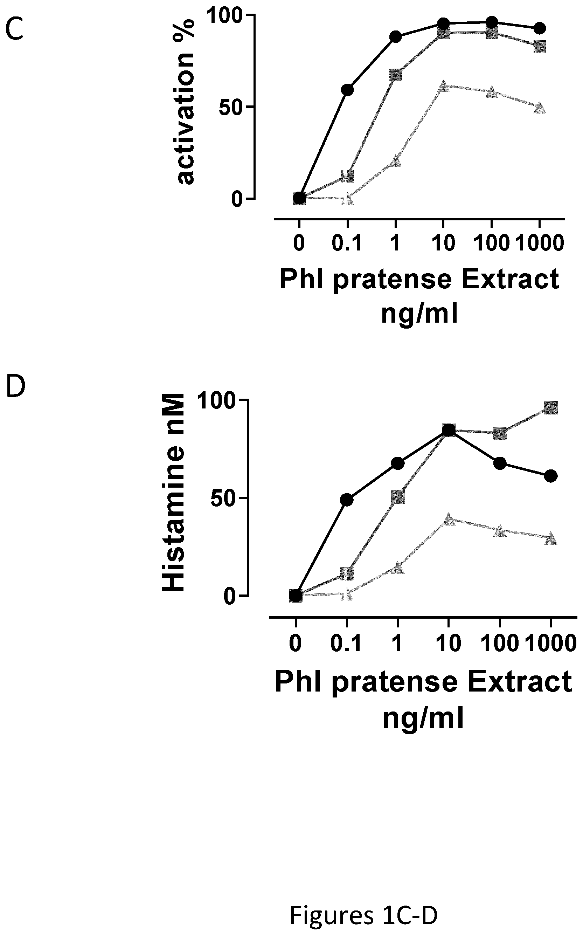

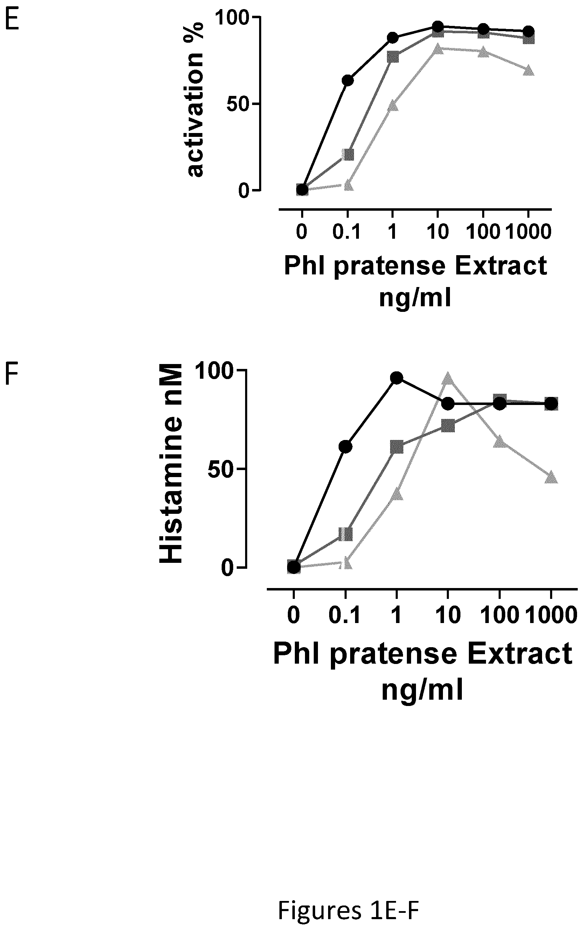

Figure 1 shows the effect of acalabrutinib on activation / degranulation of basophils after allergen-stimulation. Figures 1A, 1C and 1E show the percent activation of the cells post-treated with a BTKi (grey square=1000 nM, grey triangle = 5000nM) or control (black dot=water) at 1, 5, and 15, minutes respectively after allergen stimulation. Figures 1B, 1D, and 1F, show histamine release of the basophils post-treated with a BTKi or control at 1, 5, and 15 minutes after allergen stimulation.

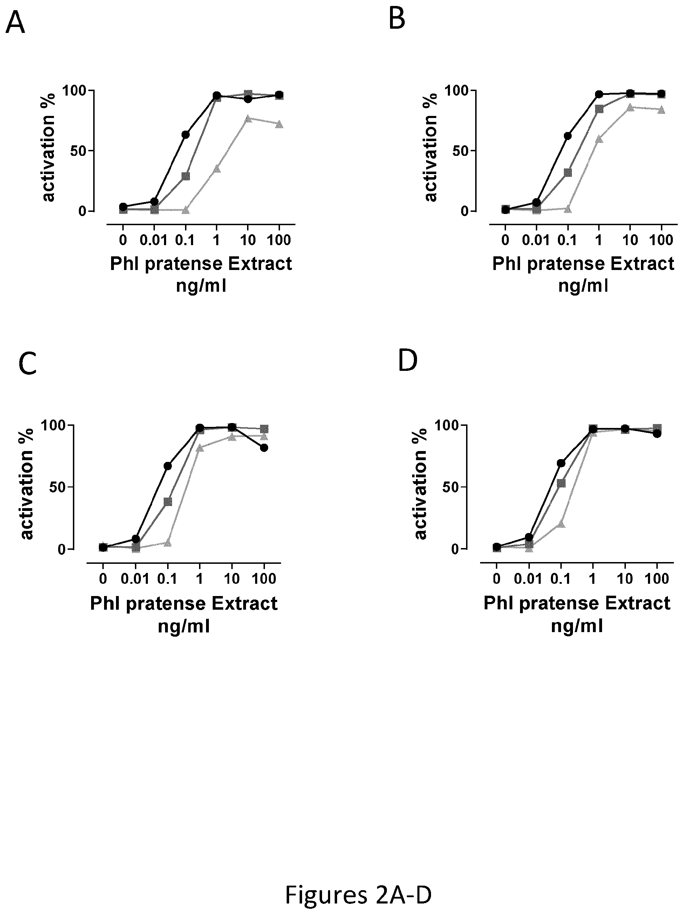

Figure 2 shows the effect of acalabrutinib on activation / degranulation of basophils after allergen-stimulation. Figure 2 shows the percent activation of the cells post-treated with a BTKi or control (grey square=1000 nM, grey triangle = 5000nM, black dot=water) at fig 2a) 1, fig 2b) 5, fig 2c) 15, and fig 2d) 30 minutes after allergen stimulation. Similar to "figure 1a" but with another independent donor.

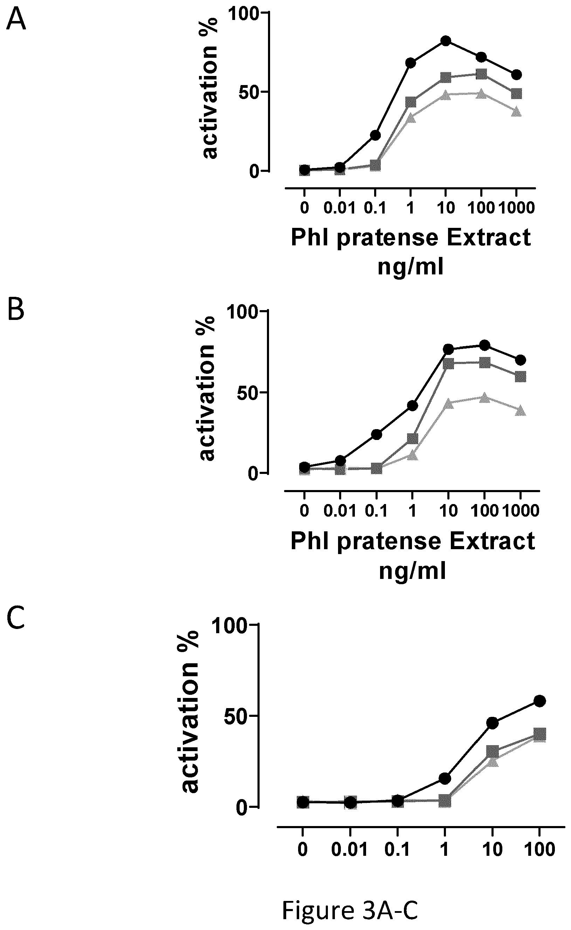

Figure 3 shows the effect of Ibrutinib on activation/ degranulation basophils after allergen-stimulation. Figures 3a-c are three independent donors and show the percent activation of the cells post-treated with BTKi or control (grey square=1000 nM, grey triangle = 5000nM, black dot=water) at five minutes after allergen stimulation.

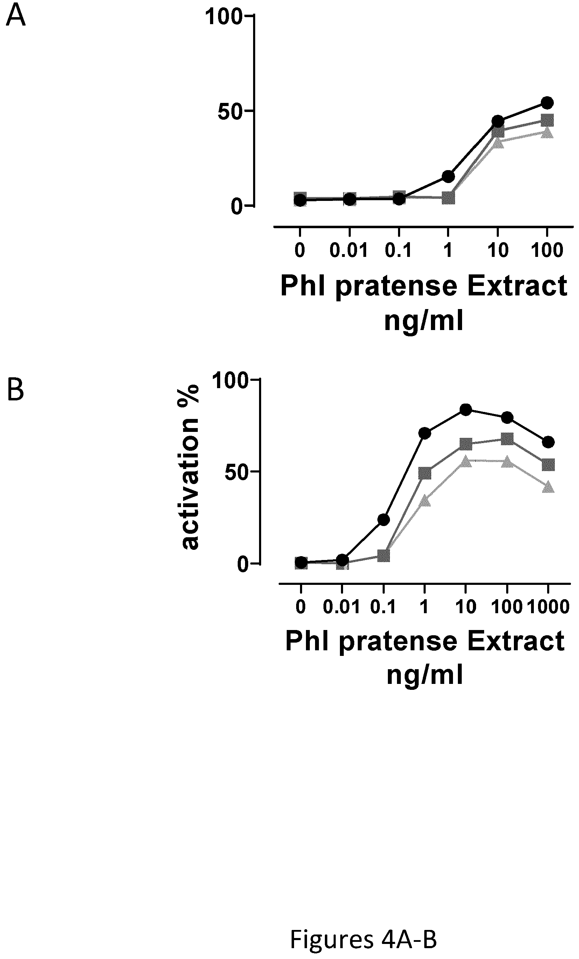

Figure 4 shows the effect of Pitrobrutinib on basophils after allergen-stimulation. Figures 4a-b are two independent donors and show the percent activation of the cells post-treated with BTKi or control (grey square=1000 nM, grey triangle = 5000nM, black dot=water) at five minutes after allergen stimulation.

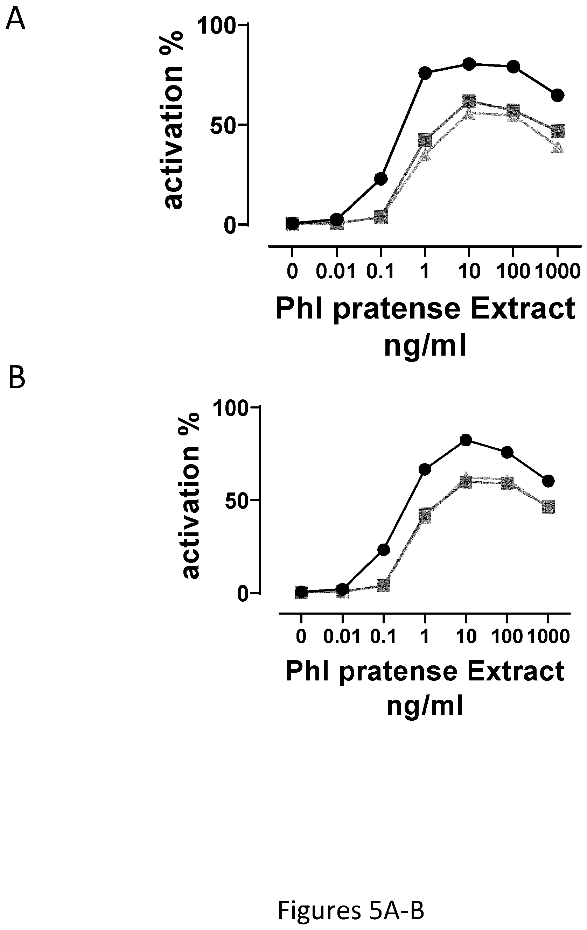

Figure 5 shows the effect of Remibrutinib (figure 5a) or Branebrutinib (figure 5b) on basophils after allergen-stimulation. Figures 5a-b show the percent activation of the cells post-treated with BTKi or control (grey square=1000 nM, grey triangle = 5000nM, black dot=water) at five minutes after allergen stimulation.

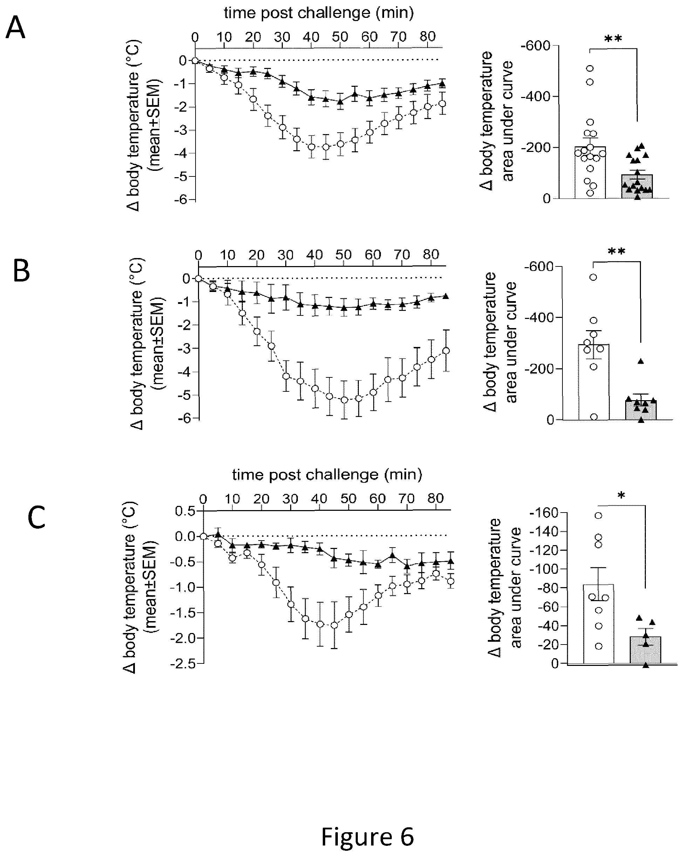

Figure 6 shows Acalabrutinib attenuates anaphylaxis severity in an active sensitisation model. C57BL/6 mice were sensitized intragastrically to peanut and challenged intraperitoneally with intragastric application of acidified water or acalabrutinib at the indicated time points. Panels show core body temperature change of mice that received water or BTKi a) four hours or b) five minutes before challenge, or c) five minutes after challenge (left) with associated area under the curve values for each mouse for statistical evaluation (right). (White circle shows water, black triangle shows BTKi). The figures showing dosing before the challenge (Figure 6a-b) are included merely as reference data.

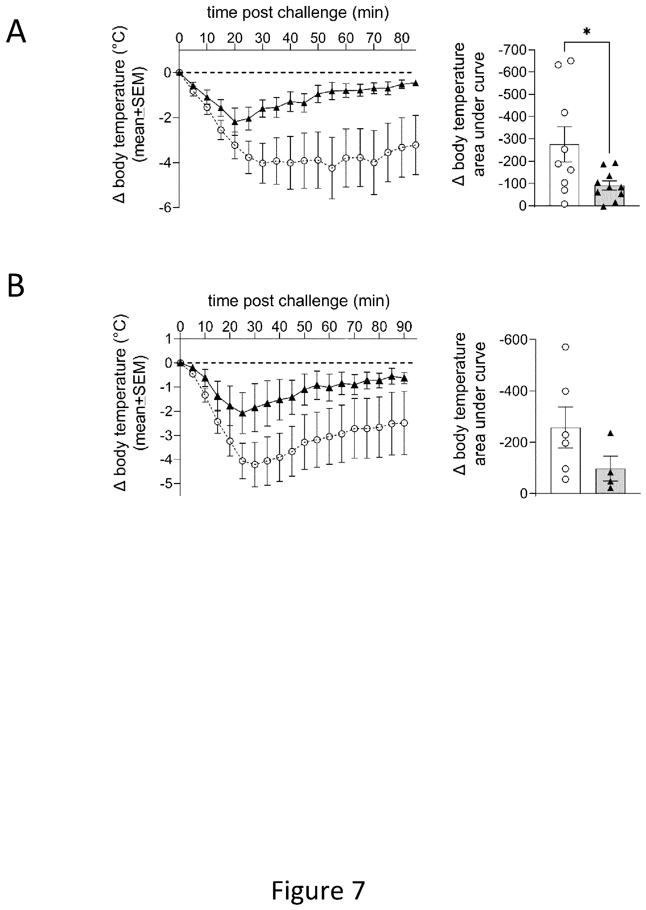

Figure 7 shows Acalabrutinib attenuates anaphylaxis severity in a passive systemic anaphylaxis model. FcεR1 transgenic mice were sensitized with human anti-Der p 2 IgE and challenged with recombinant Der p 2, both by intraperitoneal injection, and received acidified water or acalabrutinib at the indicated time points by intragastric gavage. Panels show core body temperature change of mice that received water or BTKi a) four hours or a) five minutes after challenge (left) with associated area under the curve values for each mouse for statistical evaluation. (White circle shows water, black triangle shows BTKi).

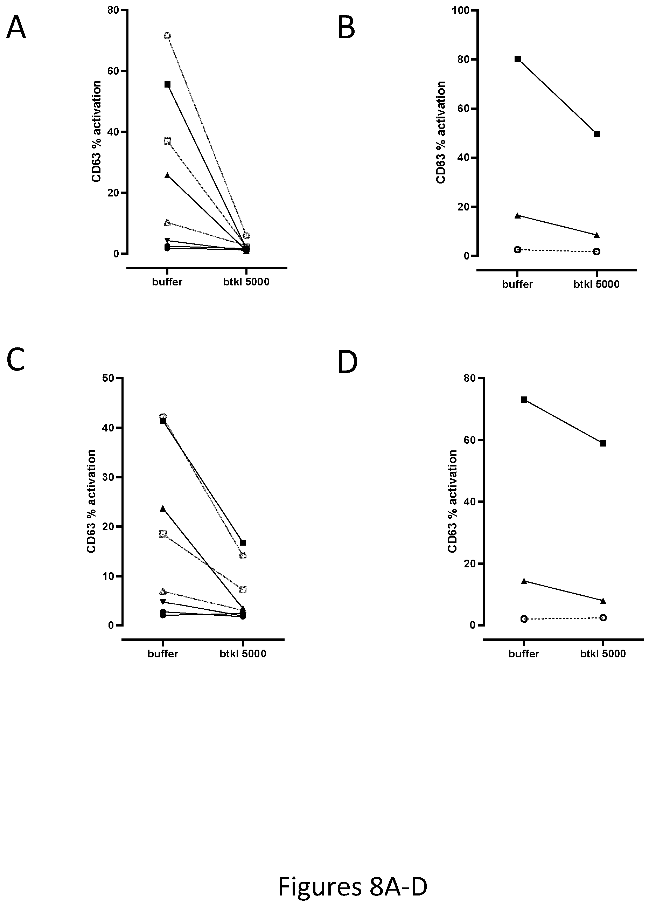

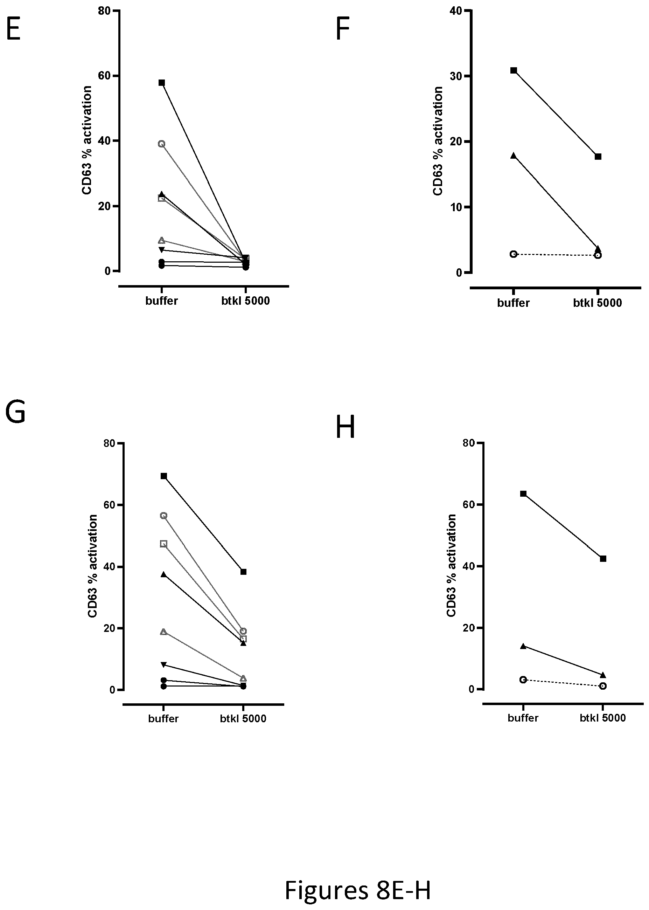

Figure 8 shows the effect of acalabrutinib on non-IgE mediated activation of Basophils. Figure 8a-b shows basophils treated with acalabrutinib 30 minutes before stimulation with either (figure 8a) buffer (black circle), Der p ex at 0.1 (black triangle, tip down), 1 (black triangle tip up) or 10ug/ml (black square) and aIgE at concentration of 10 (white triangle), 100 (white square) or 1000ug/ml (white circle), or (figure 8b) buffer (white circle), Con A at concentration of 20 (black square) or 2 ug/ml (black triangle). Figure 8c-d shows basophils treated with acalabrutinib 5 minutes after stimulation with either (figure 8c) buffer (black circle), Der p ex at 0.1 (black triangle, tip down), 1 (black triangle tip up) or 10ug/ml (black square) and aIgE at concentration of 10 (white triangle), 100 (white square) or 1000ug/ml (white circle), or (figure 8d) buffer (white circle), Con A at concentration of 20 (black square) or 2 ug/ml (black triangle). Figure 8e-f is similar to figure 8a-b and figure 8g-h is similar to figure 8c-d, but with another independent donor. The figures showing dosing before the challenge (Figure 8a-b and 8e-f) are included merely as reference data.

### DETAILED DISCLOSURE OF THE INVENTION

### Definitions

Prior to discussing the present invention in further details, the following terms and conventions will first be defined:
The term *"BTK inhibitor" (BTKi):* In the present context, the term "BTKi" refers to a compound capable of inhibiting or decreasing the kinase activity of the enzyme Bruton's tyrosine kinase (BTK). Kinase activity can be measured using a "kinase-activity assay", for example as described further in the example section herein. A "kinase activity assay" measures the ability of a protein kinase (in here BTK) to transfer a phosphate of ATP to a tyrosine residue on a substrate. A BTKi is a molecule that decreases the kinase activity of BTK in the above-mentioned activity assay, compared to a negative control, which may be an inhibitor of another kinase. The activity of a BTKi is measured by a IC₅₀ value in molar, micromolar, or nano-molar concentrations. According to their mechanism of action and binding mode, BTKis can be classified into two types: (i) irreversible inhibitors characterized by a Michael acceptor moiety able to form a covalent bond with the conserved Cys481 residue in the ATP binding site; or (ii) reversible inhibitors that bind to a specific pocket in the SH3 domain through weak, reversible interactions (e.g., hydrogen bonds or hydrophobic interactions), Brullo et al. (2021). The publications authored by Brullo et al. (2021) and Estupiñán et al. (2021), respectively, provide a comprehensive review of BTKis and their mechanism of action, kinase selectivity's and adverse events. A BTKi might be an irreversible inhibitor or a reversible inhibitor of the kinase activity. An irreversible BTKi typically binds covalently to the enzyme and inactivates the enzyme continuously. Table 1 herein lists several BTKi in pre-clinical development, clinical development or marketed.

The phrases *"an agent able to activate mast cells and*/*or basophils"* and *"an agent triggering or causing mast cell and*/*or basophil degranulation"* are used interchangeable herein and are meant to encompass a diverse group of agents able to activate and/or trigger mast cell and/or basophil degranulation, most preferably mast cell degranulation. Such agents may activate or trigger degranulation of such cells either via IgE-mediated events or non-IgE mediated events. Typically examples of agents able to do IgE-mediated activation or degranulation are allergens and agents able to trigger degranulation via non-IgE-mediated events are for example drugs, triggering the complement system or G-protein coupled receptors found on the surface of mast cells and basophils.

*Emergency treatment:* In the present context the term "emergency treatment" is defined as a condition, wherein the patient requires immediate medical attention like situations where epinephrine is required. Further, emergency treatment is life-saving treatment.

The phrase *"treatment of an allergic reaction"* is meant to encompass that the clinical signs or symptoms of an allergic reaction, including those of a severe allergic reaction, anaphylaxis or anaphylactic shock as described herein are reduced, alleviated, attenuated, postponed or even prevented upon administering a BTKi. The allergic reaction may be a Type-I hypersensitivity type.

The term *"on-going allergic reaction"* is meant to encompass an event where the degranulation of mast cells and/or basophils has resulted in the subject developing clinical sign or symptoms of an allergic reaction and that these signs have not disappeared.

In the present context, the term "Type-I allergic reaction" or *Type-I hypersensitivity* is meant to encompass an allergic reaction mediated by immunoglobulin E antibodies (IgE).

The phrase *"treatment is initiated after the allergic subject has been exposed to the antigen, such as an allergen, causing the allergic reaction"* is meant to encompass that an initial dose (e.g., a first dose) of a BTKi is administered subsequently to an event, where the allergic subject has been exposed to an antigen, such as an allergen, which may then cause or earlier has caused an allergic reaction in the subject. Typically, this event may include accidental exposure to an allergen, such as by food ingestion, by medical treatment or by stinging insects. At the time of administering the initial dose, the allergic subject may have or may not have experienced clinical signs or symptoms of the allergic reaction.

The phrase *"treatment is initiated after the allergic subject has experienced the first sign or symptoms of the allergic reaction caused by allergen exposure"* is meant to encompass that an initial dose (e.g., a first dose) of a BTKi is administered subsequently to the allergic subject had experienced clinical signs or symptoms of the allergic reaction caused by an allergen exposure. Typically, this situation may arise either upon accidental exposure to an allergen, or during continuous allergen exposure, such as during a pollen season, but where the allergic subject suddenly experiences a more severe allergic reaction, including anaphylaxis.

In the present context, the phrase *"antigen, such as allergen, causing the allergic reaction"* is meant to encompass that the allergic reaction to be treated is causally linked to the exposure of the antigen, such as the allergen, and the allergic reaction might have developed to its fully extent unless treated with a BTKi.

In the present context the phrase *"exposure (in the alternative: exposed) to an agent able to activate or trigger mast cell and*/*or basophil degranulation, e.g., an allergen"* is meant to designate that the subject in need thereof, e.g. the allergic subject, is in contact with said agent or allergen in a manner that usually would elicit said degranulation and/or any allergic response deriving therefrom. Typically, this would include exposure via inhalation, via skin, via ingestion, via stinging by insects, or via routes used for administering medicine (e.g., i.v., i.m., peroral etc.).

### Purpose of BTKi administration

An allergic reaction is a potentially life-threatening systemic allergic reaction triggered by exposure to a wide variety of allergens present in foods, medications, plants, venoms etc. The reaction following allergen exposure can vary from mild, where no treatment is necessary, to highly severe allergic reactions, including anaphylaxis, which needs emergency treatment to ensure survival of the patient.

Most allergic reactions are triggered by an unexpected or unknown exposure to an offending allergen. In food allergy, it can be highly difficult to know if one is exposed to an offending allergen as even sporadic amounts of food allergens in a food product may cause a severe allergic reaction is some subjects. For insect stings, it is even less predictable. Thus, preventing an allergic reaction following accidental exposure to a food allergen or insect stings can be highly difficult. Therefore, it is of high importance to be able to treat an acute allergic reaction, such as an ongoing allergic reaction, which starts after exposure, such as after an episodic exposure, to the allergen, such as after accidental exposure to an allergen.

A BTKi may be administered for the purpose of reducing, inhibiting, alleviating, blocking, or preventing mast cell and/or basophil degranulation and thereby reducing, inhibiting, alleviating, blocking, or preventing the development of an allergic reaction or clinical sign or symptoms of an allergic reaction to develop.

Methods and uses described herein relates to reducing degranulation of mast cells and/or basophils triggered by a subject's exposure to an agent able to trigger said degranulation by administering a therapeutically effective dose of a BTKi to said subject. In other words, methods and uses described herein relates to a BTKi administered in a therapeutically effective dose, wherein the BTKi is reducing degranulation of mast cells and/or basophils triggered by a subject's exposure to an agent able to trigger said degranulation. Consequently, methods and uses described herein relates to treating, reducing, alleviating, blocking or preventing the development of an allergic reaction or clinical sign or symptoms of an allergic reaction that is triggered by the subject's exposure to an agent able to trigger said allergic reaction, i.e. an agent able to trigger degranulation of mast cells and/or basophils which develops into said allergic response/ allergic reaction. Various aspects (methods and uses) of the present invention are listed in the section "summary of inventions" and include BTKi administering to a subject in need thereof for various purposes, such as for i) alleviating an allergic reaction, such as for alleviating an acute allergic reaction, the allergic reaction might optionally be a severe allergic reaction or anaphylaxis; ii) preventing an ongoing allergic reaction and/or ongoing degranulation of mast cells and/or basophils to progress into a severe allergic reaction or anaphylaxis; iii) mitigating the risk of developing anaphylaxis; iv) mitigation the risk of developing a delayed allergic reaction or anaphylaxis, such as biphasic anaphylaxis; v) emergency treatment of a subject who has accidentally been exposed to an agent causing or triggering mast cell and/or basophil degranulation; vi) preventing, postponing or reducing the need for epinephrine administration and/or second line treatment with antihistamine and/or corticosteroids.

In some interesting embodiments, the BTKi administration is for alleviating an allergic reaction, which allergic reaction has developed due to the subject's accidental or sudden exposure to an agent causing or triggering mast cell and/or basophil degranulation, optionally wherein the BTKi administration is for preventing or reducing the risk that the allergic reaction will progress into a severe allergic reaction or anaphylaxis. It might be understood that the allergic reaction occurring soon after accidental exposure might be an acute allergic reaction.

Typically, the allergic reaction is a Type-I allergic reaction such as a severe Type-I mediated allergic reaction and/or anaphylaxis.

It should be understood that in connection with treatment of a severe allergic reaction, such as anaphylaxis, the administration of the BTKi might reduce the need of epinephrine treatment and/or second-line treatment with antihistamines or steroids.

Epinephrine treatment is today the first line of treatment if a human is experiencing a severe allergic reaction including anaphylaxis, whereas antihistamines and glucocorticoids are considered solely second-line therapy. Antihistamines, having a slow onset of action, are unable to stabilize or prevent mast cell degranulation, and unable to target other mediators of the severe allergic reaction than histamine. Further, antihistamines will not effectively treat cardiovascular and respiratory symptoms such as hypotension or bronchospasm.

Therefore, there is an unmet need for alternative options or adjunctive options for first-line treatment as well as second-line treatment of severe allergic reactions, including anaphylaxis. Therefore, in one embodiment, the BTKi might be administered before and/or after and/or concurrently with epinephrine treatment. Thus, the BTKi might be used as a combination therapy with epinephrine. Preferably, the BTKi may be administered before the epinephrine treatment.

In another embodiment, administration of the BTKi reduces the need of epinephrine treatment and/or second-line treatment with antihistamine or steroid.

In a further or alternative embodiment, the BTKi may be administered as a second-line drug.

Thus, the administration of the BTKi may replace i.v. antihistamine or it may be administered in combination with antihistamine treatment.

### Timing of BTKi administration

Various aspects of the invention are listed in the section "summary of inventions" and a common key feature of all the aspects relates to the timing of a subject's administration of a BTKi inhibitor in that the present treatment concept is mostly intended to be for acute treatment, episodic treatment and/or emergency treatment of subjects in need thereof. A dose of the BTKi may be administered within a period close to the subject's exposure to an agent able to trigger degranulation of mast cells and/or basophils (e.g., allergen) and/or close to the initiation of mast cell degranulation following said mast cell degranulation, i.e. as presently claimed. As the BTKi has been shown to produce a fast prompt response in reducing degranulation, even when degranulation has started (is ongoing), the BTKi administration might be performed at or after the subject's exposure to the offending agent.

In one embodiment, the timing of the administration of a BTKi inhibitor is related to the point in time that the subject was exposed to the agent able to trigger the degranulation.

In a further embodiment or alternative embodiment, the timing of the administration of a BTKi inhibitor is related to the point in time that the mast cells and/or basophils are activated and/or has initiated degranulation in consequence of said exposure.

In a still further embodiment or alternative embodiment, the timing of the administration of a BTKi inhibitor is related to the point in time that the subject is exhibiting a clinical sign or symptom of an allergic reaction, which has developed in consequence of said degranulation or exposure.

Preferably, the clinical sign or symptom of an allergic reaction will be the first sign or symptom on an allergic reaction after the most recent exposure or degranulation. That is to say that an ongoing allergic reaction has occurred in consequence of the exposure/degranulation.

Therefore, a dose of the BTKi is administered within a period ranging from the subject's exposure to the agent triggering said degranulation until 24 hours after said exposure, but preferably prior to or when the subject is exhibiting a clinical sign or symptom of an allergic reaction which has developed in consequence of said degranulation or exposure.

That is to say that a dose of BTKi may be administered after the subject's exposure to the offending agent within a period not exceeding 24 hours and/or prior to or when the subject in need thereof has experienced a clinical sign or symptoms, such as a first clinical sign or symptoms, of an allergic response developed in response to said degranulation or exposure, with the notion that the BTKi is still administered within the 24 hours from exposure.

In more specific embodiments, where the method is for alleviation an allergic reaction, such as an acute allergic reaction, the BTKi may be administered within a period ranging from the subject's exposure to an allergen and until 24 hours after said exposure, but preferably prior to or when the subject is exhibiting a clinical sign or symptom of an allergic reaction which has developed in consequence of said degranulation or exposure. In such embodiments, it might be understood that the allergen is able to trigger mast cell and/or basophil degranulation in said subject and/or developing an allergic reaction in consequence of said granulation or exposure.

In some embodiments, a BTKi might be administered after the subject is exhibiting a clinical sign or symptom of an allergic reaction, but prior to the progression of said allergic reaction into a severe allergic reaction or anaphylaxis.

That is to say that in some embodiments, a dose of BTKi may be administered within a period ranging from the subject's exposure to the agent triggering said degranulation until 24 hours after said exposure, but preferably prior to the subject is exhibiting a clinical sign or symptom of a severe allergic reaction or anaphylaxis, which has developed in consequence of said degranulation or exposure.

Thus, as general concept, the treatment (i.e. the administration of the BTKi) might be initiated as follows: i) after the subject in need thereof (e.g., the allergic subject) has been exposed to the agent able to trigger mast cell and/or basophil degranulation in said subject (e.g. an allergen); ii) prior to, at or following the subject is experiencing a clinical sign or symptom of an allergic reaction which has developed in consequence of said degranulation or exposure or iii) prior to the subject is experiencing a clinical sign or symptom of a severe allergic reaction which has developed in consequence of said degranulation or exposure.

Therefore, it can be summarised that the dose of a BTKi is administered within a period ranging from the subject's exposure to the agent triggering said degranulation until 24 hours after said exposure, but preferably prior to the subject is exhibiting a clinical sign or symptom of an allergic reaction, a severe allergic reaction or anaphylaxis, which has developed in consequence of said degranulation or exposure.

It might be understood that the subject's exposure to the agent able to trigger the degranulation in said subject (e.g., an allergen) refers to the most recent exposure, an episodic exposure or an accidental exposure. Thus, the exposure might not include chronic or extended exposure to an offending agent (e.g., allergen).

As mentioned, a BTKi may be administered within a period ranging from the subject's exposure to the agent triggering said degranulation until 24 hours after said exposure. However, in some embodiments, the period for performing a single dose administration, optionally a repeat dose, is preferably until 20 hours, more preferably until 15 hours, such as until 12 hours, such as until 10 hours after said exposure.

As the subject's exposure to the offending agent may develop quickly into an allergic reaction, a dose of BTKi may be administered to said subject within 20 hours, 15 hours or 10 hours from the subject's exposure to the agent able to triggering said degranulation, preferably within 9 hrs, 8 hrs, 7hrs, 6 hrs, 5 hrs, 4 hrs, 3 hrs, 2.5 hrs, 2 hrs, 1.5 hr, 1 hr, 50 min, 40 min, 30 min, 20 min, 15 min, 10 min or 5 min.

In particularly, it is envisaged that a dose of BTKi is administered to said subject within 40 min, preferably within 30 min, more preferably within 20 min, such as within 15 min, such as within 10 min from the subject's exposure to the offending agent. More preferably, a dose of BTKi is administered to said subject within 20 min, such as within 15 min, such as within 10 min from the subject's exposure to the offending agent.

While there might be some lack time between exposure to the offending agent and the initiation of mast cell and/or basophil degranulation, the administration of a BTKi might be within 40 min after initiation of said degranulation, preferably within 30 min, such as 25 min, 20 min, 15 min or 10 min.

The allergic reaction referred to herein is typically a Type-I allergic reaction, which refers to an allergic reaction mediated by allergen-specific IgE antibodies produced by the immune system in response to allergens. As mentioned, the cross-linking of such IgE-antibodies bound to the FcεRI at the surface mast cells and basophils by allergens initiates an allergic reaction following allergen exposure. Thus, the treatment of an allergic reaction as described herein may further be defined as the treatment of a Type-I allergic reaction, which in the allergic subject would be triggered following exposure to an allergen that the allergic subject is sensitised to. Accordingly, the allergic subject has IgE antibodies raised against the allergen.

Advantageously, in the methods and uses described herein the purpose or outcome of the BTKi administration is that the progression of an allergic reaction, such as a mild allergic reaction might be prevented from developing into a more severe allergic reaction or anaphylaxis. It is considered that the BTKi may be administered soon after exposure as described above, but the subject in need thereof might also seek relief when the subject is exhibiting an allergic reaction, preferably a mild allergic reaction, which could progress into a more severe reaction.

Thus, in some embodiments, a dose of BTKi is administered within 10 hours after the subject is exhibiting an allergic reaction in consequence of being exposed to an agent able to trigger the degranulation, preferably dose of BTKi is administered within 9 hrs, 8 hrs, 7hrs, 6 hrs, 5 hrs, 4 hrs, 3 hrs, 2.5 hrs, 2 hrs, 1.5 hr, 1 hr, 50 min, 40 min, 30 min, 20 min, 15 min, 10 min or 5 min. Preferred time points are within 30 min, such as 25 min, 20 min, 15 min or 10 min.

Following exposure to an agent able to trigger mast cell and/or basophil degranulation, such as an allergen, the immune system will start to respond and develop an allergic condition, which is called allergic inflammation.

There are two phases of in the basic process of allergic inflammation: The induction phase (sensitisation) and the effector phase.

The induction phase involves antigen presenting cells (APCs), T cells, Th2 cytokines, such as interleukin (IL)-4, IL-5 and IL-13, class switching of B cells, IgE secretion and binding to the high-affinity IgE receptor FcεRI on the membrane of mast cells and basophils, forming sensitized mast cells and basophils.

The effector phase begins when same allergen as in the induction phase, cross-links two adjacent IgEs on sensitized mast cells or basophils. Activated mast cells and/or basophils subsequently degranulate, meaning the release of proinflammatory mediators or cytokines, thereby causing the clinical manifestations of allergy.

Overall, this means that a subject in the induction phase, will not show any signs or symptoms of an allergic reaction, and thus, will not be in the need of any treatment.

### Subject in need thereof

The term "a *subject in need thereof"* is meant to encompass an animal with mast cell / basophil degranulation mediated via the BTKi pathway. Such subjects are preferably humans, including humans of all ages (such as children, adolescent and adult), or a domestic animal, such as a horse, dog or cat.

In one embodiment, the subject is an allergic subject. The term *"allergic subject"* is meant to encompass that the subject is sensitised to an antigen, such as an allergen. The sensitisation to one or more allergens can be measured by use of a skin prick test, wherein small amounts of allergen is administered just below the skin surface. The development of a "wheal" (a raised, red, itchy bump and surrounding "flare") indicates sensitivity to the given allergen. Additionally, the presence of IgE antibodies to an allergen can be detected using conventional immunoassay methods such as for example Immunocap (Thermo Scientific), microarrays or other methods, wherein allergen specific IgE is detected and/or measured. When using an Immunocap method, a detection of IgE at a level above 0.35kU/L in a biological sample (e.g., blood serum) is typically associated with atopic disease or allergy.

A subject in need thereof has been exposed, such as just exposed to an agent able to trigger mast cell and/or basophil degranulation in said subject. Exposure might be unintentionally, i.e, accidentally.

In some embodiments thereof, the subject may be diagnosed with an allergy (i.e. being an allergic subject) towards the agent triggering said degranulation and/or allergic reaction and/or anaphylactic shock.

In some embodiments thereof, the subject may be susceptible to develop an allergic reaction, such as a severe allergic reaction or anaphylaxis when exposed to the agent able to trigger the degranulation in said subject.

In further embodiments, the subject in need thereof is in risk of developing a severe allergic reaction and/or anaphylactic shock. Such subjects may have a history of anaphylaxis and might have had need of epinephrine treatment at a prior exposure.

In another embodiment, the subject has a risk of developing a delayed severe allergic reaction and/or anaphylactic shock.

In a further embodiment, the subject is diagnosed with an allergy towards the allergen triggering the degranulation of the basophils and/or mast cells and/or allergic reaction and/or anaphylactic shock.

In still further embodiments, the subject is accidentally exposed to an allergen.

The allergic subjects can be divided into different types depending on their history of allergy.

One group of subjects might not have previously experienced clinical signs or symptoms of an allergic reaction, including severe allergic reaction or anaphylaxis. Another group of subjects might have a history of atopy or allergic reactions in the past. Such subjects might have been diagnosed with allergy, a severe allergy and/or anaphylaxis. Typically, the subject in need of treatment has previously been diagnosed with anaphylaxis or might be susceptible to anaphylaxis when exposed to an allergen. Anaphylaxis might be diagnosed by determining the level of serum tryptase subsequent to an anaphylactic episode. Therefore, an allergic subject in need of BTKi treatment as described herein may earlier have been diagnosed with elevated levels of serum tryptase.

The presence of atopy in a subject is associated with an increased risk of developing one or more of the atopic diseases or conditions such as atopic dermatitis, asthma, allergic rhinitis, allergic conjunctivitis or allergic rhino-conjunctivitis, food allergy, anaphylaxis, anaphylactic shock, allergic bronchopulmonary aspergillosis, urticaria, itch and hives.

Therefore, in some embodiments, the allergic subjects have or have been diagnosed with an atopic disease as mentioned above. In some embodiments, the subject is, or has previously been, diagnosed with and/or experienced symptoms of allergy against one or more allergens. Thus, the subject is, or has previously been, diagnosed with allergy by detection or measurement of specific IgE to one or more allergens, and/or by a positive skin prick test using one or more allergens. In some, more specific embodiments, the subject is or has been diagnosed with a pollen allergy, a food allergy, an insect venom allergy and/or allergy to medicine. Typically, the allergic subject has a food allergy or an insect venom allergy.

### Agent able to trigger mast cell and/or degranulation

The mast cell and/or basophil degranulation and/or allergic reaction to be treated as described herein may be triggered by an agent able to trigger Type-I mediated hypersensitivity, such as an allergen, or alternatively by an agent able to trigger non-IgE mediated activation of mast cells/ basophils, such oversulfated heparin, aspirin and other NSAIDs, antibiotics, including vancomycin and the fluoroquinilones, opiates, and drugs used in general anesthesia, particularly NMBAs (Cianferoni 2021). For example, are Concavalin A able to activate basophils by induction of the JAK2/STAT3 pathway, which the FDA approved BTKi, ibrutinib, is able to inhibit (Zhang and Qiu 2019). Further, as demonstrated in example 1, it was possible to reduce the Concavalin A induced basophil activation using acalabrutinib.

In some embodiments, the allergen may be any one of a pollen allergen, a mite allergen, a food allergen, an insect venom allergen, and/or a medicine. Typical examples of pollen allergen may be from grass pollen, weed pollen (e.g., ragweed pollen or mug wort pollen) or tree pollen (e.g., birch pollen, hazel pollen, alder pollen, hornbeam pollen, beech pollen, oak pollen, cedar pollen, ash pollen, and olive pollen). Typical examples of mite allergens are from house dust mites *(Dermatophagoides farinae, Dermatophagoides pterinussinus,* and *Blomia tropicalis).* Typical examples of animal allergens might include those from a cat, a dog or a horse. Typical examples of food allergens may be selected from the group consisting of soy, wheat, eggs, peanuts, tree nuts, sesame, milk, fish, shellfish, mushroom, and stone fruits. Typical examples of insect venom allergens are those from wasp and bee stings.

Typical examples on medicine potentially causing an allergic Type-I reaction may be selected from the group consisting of antibiotics (e.g., amoxicillin, ampicillin, penicillin, tetracycline), nonsteroidal anti-inflammatory drugs (e.g., ibuprofen and naproxen), chemotherapy drugs, monoclonal antibody therapy (e.g., cetuximab, rituximab), insulin, antiseizure drugs (e.g., carbamazepine, lamotrigine, phenytoin)

### Clinical signs and symptoms, and allergic reactions

The overall purpose of administering a BTKi is to inhibit and/or stop and/or block degranulation of basophils and/or mast cells just after the basophils and/or mast cells have been activated by the exposure to a triggering allergen. In embodiments thereof, the BTKi is administered prior to or when the subject is exhibiting a clinical sign or symptom of an allergic reaction, severe allergic reaction, or anaphylaxis in consequence of said degranulation.

This allergic reactions/conditions or anaphylaxis as described above are manifested in several clinical signs and symptoms, which might be grated according to severity, from mild to fatal. The allergic reactions might be on a continuum from mild to fatal and the importance of early treatment to prevent progression into a severe state is key.

However, clinical signs and symptoms may not show immediately at or subsequently to the allergen exposure but can be delayed dependent on the immunological reaction.

Thus, in one embodiment, the administration of the BTKi may be initiated before the subject is exhibiting a clinical sign or symptom of the allergic reaction/condition.

In response to allergen exposure, clinical signs and symptoms start to develop on the affected subject. The signs and symptoms can be divided into groups dependent on the part of the body involved. For each of the groups, several symptoms can be described. The symptoms might be divided into mild, moderate and severe, depending on how much they affect the subject. Typically, the clinical signs or symptoms of an allergic reaction such as a Type-I allergic reaction include one or more of the following groups:
Cardiovascular symptoms relate to conditions affecting the heart or blood vessels. It may be presented as mild symptoms like sense of weakness, sense of dizziness, pre-syncope. Moderate symptoms are hypotension, syncope. Severe symptoms are cardiac arrest.

Neurologic symptoms relate to conditions affecting the nervous system. Herein mild symptoms which are sense of confusion, sense of drowsiness, sense of impending doom. Moderate symptoms are GCS (Glasgow Coma Scale) 13-14 and severe is GSC<13. The GCS' three groups for examining the patient as outlined below:
- Eye response
   ∘ to verbal command (+3)
   ∘ to pain (+2)
   ∘ no eye opening (+1)
- Verbal response
   ∘ Oriented (+5)
   ∘ Confused (+4)
   ∘ Inappropriate words (+3)
   ∘ Incomprehensible sounds (+2)
   ∘ No verbal response (+1)
- Motor response
   ∘ Obeys commands (+6)
   ∘ Localised pain (+5)
   ∘ Withdrawal from pain (+4)
   ∘ Flexion to pain (+3)
   ∘ No motor response (+1)

A sum of one number from each group makes up the final score.

Respiratory symptoms relate to conditions affecting the part of the body involved in gas exchange, including the lungs, nose, trachea and breathing muscles. Here mild symptoms are chest tightness, dyspnea, new onset cough, throat tightness or discomfort, voice change and wheezing. Moderate symptoms are new onset of persistent cough, increased work of breathing (WOB), stridor and hypoxemia. Severe symptoms are respiratory failure, reduced blood pressure of less than 90mm Hg or greater than 30% decrease form baseline.

Mucosal symptoms relate to the inner lining of some organs and body cavities, such as nose, mouth, lungs, and stomach /angioedema. Mild symptoms are mouth tingling, itchy mouth or throat, metallic taste, facial swelling, conjunctival injection, chemosis, nasal congestion, rhinorrhea, throat clearing, lip swelling, mild tongue, soft palate and/or uvula swelling. Moderate symptoms are drooling, moderate tongue swelling, soft palate and/or uvula swelling. Severe symptoms are severe swelling of the tongue, soft palate and/or uvula.

Skin symptoms encompass mild symptoms such as skin pruritus, skin discomfort, occasional skin scratching (itching/pruritis), localised skin scratching or excoriations, localised skin urticaria (hives), localised skin erythema, flushed skin, pale skin, and moderate symptoms, such as continuous skin scratching, generalised skin scratching (itching/pruritis) or excoriations, generalised skin urticaria a, skin flushing and generalised skin erythema.

Gastrointestinal (GI) symptoms relate to mild symptoms, such as nausea, abdominal pain, emesis, vomiting and diarrhoea and moderate symptoms, such as frequent or continuous nausea or abdominal pain, crampy abdominal pain, sense of being distressed due to GI symptoms.

The above-mentioned symptoms are exemplifications of allergic reactions, and the present invention is not limited to the listed signs and symptoms.

In one embodiment, said clinical signs or symptoms are mild clinical signs and symptoms selected from the list consisting of sense of weakness, sense of dizziness, pre-syncope, sense of confusion, sense of drowsiness, sense of impending doom, chest tightness, dyspnea, new onset cough, throat tightness or discomfort, voice change and wheezing, mouth tingling, itchy mouth or throat, metallic taste, facial swelling, conjunctival injection, chemosis, nasal congestion, rhinorrhea, throat clearing, lip swelling, mild tongue, soft palate and/or uvula swelling, skin pruritus, skin discomfort, occasional skin scratching (itching/pruritis), localised skin scratching or excoriations, localised skin urticaria, localised skin erythema flushed skin, pale skin, nausea, abdominal pain, emesis, vomiting and diarrhoea.

In another embodiment, said clinical signs and symptoms are moderate clinical signs and symptoms selected from the list consisting of hypotension, syncope, GCS 13-14, new onset of persistent cough, increased work of breathing (WOB), stridor and hypoxemia, drooling, moderate tongue swelling, soft palate and/or uvula swelling, continuous skin scratching (itching/pruritis), generalised skin scratching (itching/pruritis or excoriations, generalised skin urticaria a, skin flushing and generalised skin erythema, frequent or continuous nausea or abdominal pain and sense of being distressed due to GI symptoms.

In a further embodiment, said clinical signs and symptoms are severe clinical signs and symptoms selected from the list consisting of cardiac arrest, respiratory failure, severe swelling of the tongue, soft palate and/or uvula.

The manifestation of the different symptoms in a subject might be used to clinically grade the severity of the allergic reaction using the grading system described by Dribin TE. et al. (2021). This grading system uses the most severe symptom present at a given subject for determining the grading. The system divides acute allergic reactions into five stages depending on the symptoms, where five is the most severe.
Stage 1: Mild reaction of "a-c"
   a. Skin (e.g., Localised urticaria, erythema)
   b. Gastrointestinal (e.g., 1-2 episodes of emesis or diarrhoea)
   c. Mucosal/angioedema (e.g., Facial swelling, rhinorrhea)
Stage 2: Two or more of the mild symptoms or 1 of the moderate symptoms of "d-f"
   d. Skin (e.g., *Mild:* Localised urticaria, erythema / *Moderate:* generalised urticaria, erythema)
   e. Gastrointestinal (e.g., *Mild:* 1-2 episodes of emesis/diarrhoea or *Moderate* >3 episodes of emesis/diarrhoea)
   f. Mucosal/angioedema (e.g., *Mild:* facial swelling, rhinorrhea or *Moderate:* moderate oropharyngeal swelling)
Stage 3: Any moderate of "g-i"
   g. Cardiovascular (e.g., weak, dizzy, palpitations)
   h. Neurologic (e.g., confusion, drowsy)
   i. Respiratory (e.g., dyspnea, chest tightness, new unset cough, wheezing w/o increased WOB
Stage 4: Any moderate "j-l" or severe "m"
   j. Cardiovascular (e.g., hypertension, syncope)
   k. Neurologic (e.g., GSC 13-14)
   l. Respiratory (e.g., new onset persistent cough, hypoxemia, increased WPB (+/- wheezing), stridor w/o increased WOB
   m. Mucosal/angioedema (e.g., severe swelling of oropharyngeal /tongue/palate/uvula)
Stage 5: Any severe "n-p"
   n. Cardiovascular (e.g., anaphylactic shock, cardiac arrest)
   o. Neurological (e.g., Glasgow Coma Scale)
   p. Respiratory

Anaphylaxis is a subgroup of severe allergic reactions. It is an acute potential life threatening systemic allergic reaction that occurs suddenly after contact with an allergy causing substance. Anaphylaxis is a definition used in the clinic to describe a patient with a severe acute allergic reaction. There is no worldwide consistency on how to diagnose anaphylaxis (Adriana et al. 2022). One of the broadest accepted diagnostic tools is developed by National Institute of Allergy and Infectious Disease (NIAID) and Food Allergy and Anaphylaxis Network (FAAN) symposium in 2006 (Sampson et al. 2006).

The symposium defined anaphylaxis in its broadest meaning as *"a serious allergic reaction that is rapid in onset and may cause death".*

According to NIAID and FAAN, anaphylaxis is highly likely when one of the following three criteria are fulfilled:
1. Acute onset of an illness (minutes to several hours) with involvement of the skin mucosal tissue or both (e.g., generalised hives, pruritus or flushing, swollen lips-tongue-uvula), and at least one of the following:
   a. Respiratory compromise (e.g., dyspnea, wheeze-bronchospasm, stridor, reduced peak expiratory flow, hypoxemia)
   b. Reduced blood pressure or associated symptoms of end-organ dysfunction (e.g., hypotonia, syncope, incontinence)
2. Two or more of the following that occur rapidly after exposure to a likely allergen for that patient
   a. Involvement of the skin-mucosal tissue (e.g., generalised hives, itch-flush, swollen lips-tongue-uvula)
   b. Respiratory compromise (e.g., dyspnea, wheeze-bronchospasm, stridor, reduced peak expiratory flow, hypoxemia)
   c. Reduced blood pressure or associated symptoms (e.g., hypotonia, syncope, incontinence)
   d. Persistent gastrointestinal symptoms (e.g., crampy abdominal pain, vomiting)
3. Reduced blood pressure after exposure to a known allergen for that patient.
   a. Infant and children: low systolic blood pressure (age specific) or greater that 30% decrease in systolic blood pressure
   b. Adults: systolic blood pressure less than 90mm Hg or greater than 30% decrease from that person's baseline.

In a fraction of the cases, anaphylaxis can progress into anaphylactic shock, which is the most severe state of anaphylaxis, defined as fulfilling the above-mentioned criteria for anaphylaxis combined with hypoperfusion or even cardiac arrest.

Therefore, in one embodiment, said Type-I allergic reaction is anaphylactic shock. In a further embodiment said anaphylactic shock includes at least one clinical sign or symptom which is hypotension.

Anaphylaxis and later anaphylactic shock occur as part of a continuum. Symptoms not immediately life-threatening may progress rapidly unless treated promptly. Further, the clinical presentation and severity of symptoms differ among individuals and may change over time within the same individual.

It is thus of high importance that treatment is initiated rapidly following signs of the first symptoms associated with anaphylaxis.

In one embodiment, said severe allergic reaction or anaphylaxis includes at least one clinical sign or symptom from each of two different types of clinical signs and symptoms selected from a first, a second, a third, a fourth or a fifth type of clinical signs and symptoms, wherein
a first type of clinical signs and symptoms is having skin involvement, wherein the clinical sign or symptoms is selected from the group consisting of skin pruritus, skin discomfort, occasional skin scratching (itching/pruritis), localised skin scratching or excoriations, localised skin urticaria (hives), localised skin erythema, flushed skin, pale skin, continuous skin scratching, generalised skin scratching (itching/pruritis), excoriations, generalised skin urticaria a, skin flushing and generalised skin erythema, or
a second type of clinical sign and symptoms is having mucosal tissue involvement wherein the clinical sign or symptoms is selected from the group consisting of mouth tingling, itchy mouth or throat, metallic taste, facial swelling, conjunctival injection, chemosis, nasal congestion, rhinorrhea, throat clearing, lip swelling, mild tongue, soft palate and/or uvula swelling, moderate tongue swelling, soft palate and/or uvula swelling, swelling of the tongue, soft palate and/or uvula; or
a third type of clinical signs and symptoms is respiratory compromise wherein the clinical sign or symptom is selected from the group consisting of chest tightness, dyspnea, new onset cough, throat tightness or discomfort, voice change and wheezing, new onset of persistent cough, increased WOB, stridor and hypoxemia, respiratory failure, reduced blood pressure of less than 90mm Hg or greater than 30% decrease from baseline; or
a fourth type of clinical signs and symptoms is cardiovascular compromise, wherein the clinical sign or symptom is selected from the group consisting of sense of weakness, sense of dizziness, pre-syncope. Moderate symptoms are hypotension, cardiac arrest; or
a fifth type of clinical signs and symptoms is having neurologic compromise, wherein the clinical signs and symptoms are selected from the group consisting of sense of confusion, sense of drowsiness, sense of impending doom. Moderate symptoms are GCS 13-14 and severe is GSC<13.

Even though an allergic reaction can be divided into grades of severity, it develops as part of a continuum. Symptoms not immediately life-threatening may progress rapidly unless treated promptly. Further the clinical presentation and severity of symptoms differ among individuals and may change over time within the same individual.

It is thus of high importance that treatment is initiated rapidly following signs of the first symptoms associated with anaphylaxis or severe allergic reaction.

An allergic reaction can be an acute allergic reaction when clinical signs and symptoms appear rapidly after allergen exposure. Accordingly, in one embodiment, said allergic reaction is developed as an acute response to the allergen exposure.

In a further embodiment, said method of treating an allergic reaction is a method of emergency treatment of an allergic reaction, such as a Type-I allergic reaction including anaphylaxis.

Anaphylaxis or severe allergic reactions can be subdivided into different phases, including bi-phasic, persistent, and refractory anaphylaxis.

Thus, in one embodiment, said anaphylaxis is selected from the group consisting of uniphasic anaphylaxis, bi-phatic anaphylaxis, persistent anaphylaxis, refractory anaphylaxis, and anaphylactic shock.

Bi-phasic anaphylaxis or bi-phasic severe allergic reaction is typically a condition where the initial signs or symptoms completely resolve before the onset of new symptoms. The onset of new symptoms may occur without allergen re-exposure. Further, the onset of new symptoms may occur within 1 to 48 hours from resolution of the initial symptoms.

Therefore, in one embodiment, the method is for treating a delayed allergic reaction, such as a delayed type-I allergic reaction, or a combined acute and delayed allergic reaction, such as a combined acute and delayed type-I allergic reaction. Typically, the delayed allergic reaction occurs between 2 to 48 hours after the allergic subject has been exposed to the allergen causing or triggering mast cell / basophil degranulation or the allergic reaction. Typically, the delayed allergic reaction occurs between 4 to 36 hours, such as between 6 to 30 hours after said allergen exposure.

Early onset of treatment is of high importance in relation to later development of bi-phasic anaphylaxis. Thus, in one embodiment, methods for treating an acute allergic reaction by a BTKi may also treat a bi-phasic allergic reaction, such as a severe biphasic allergic reaction. In another embodiment, the acute allergic reaction is bi-phasic anaphylaxis. In still another embodiment, said combined acute and delayed allergic reaction is biphasic anaphylaxis.

Persistent anaphylaxis or persistent severe allergic reaction may be present if symptoms or examination findings persist for a longer period, such as at least 4 hours. Thus, in one embodiment, the allergic reaction is persistent severe allergic reaction. In another embodiment, the allergic reaction is persistent anaphylaxis.

Refractory anaphylaxis or refractory severe allergic reaction is seen in cases where presence of the diagnosis (anaphylaxis or severe allergic reaction) follows appropriate adrenaline dosing or symptom-directed medical management and wherein the initial reaction is treated with 3 or more appropriate doses of adrenaline.

Thus, in one embodiment, the acute allergic reaction is refractory severe allergic reaction. In another embodiment, the allergic reaction is refractory anaphylaxis.

Like the acute allergic reaction, it is preferred to treat with the BTKi before the allergic subject presents with a clinical sign or symptom of the delayed allergic reaction.

Thus, in one embodiment, the BTKi is administered within two hours from the allergic subject presents with a clinical sign or symptom of the delayed allergic reaction, preferably wherein the treatment is initiated within 1.5 hour, such as within 1 hour, such as within 50 minutes, such as within 40 minutes, such as within 30 minutes, such as within 20 minutes, such as within 15 minutes, such as within 10 minutes, such as within 5 minutes from the allergic subject presents with a clinical sign or symptom of the delayed allergic reaction.

In one embodiment, said clinical signs or symptoms are mild clinical signs and symptoms selected from the list consisting of sense of weakness, sense of dizziness, pre-syncope, sense of confusion, sense of drowsiness, sense of impending doom, chest tightness, dyspnea, new onset cough, throat tightness or discomfort, voice change and wheezing, mouth tingling, itchy mouth or throat, metallic taste, facial swelling, conjunctival injection, chemosis, nasal congestion, rhinorrhea, throat clearing, lip swelling, mild tongue, soft palate and/or uvula swelling, skin pruritus, skin discomfort, occasional skin scratching (itching/pruritis), localised skin scratching or excoriations, localised skin urticaria, localised skin erythema flushed skin, pale skin, nausea, abdominal pain, emesis, vomiting and diarrhoea.

In another embodiment, said clinical signs and symptoms are moderate clinical signs and symptoms selected from the list consisting of hypotension, syncope, GCS 13-14, new onset of persistent cough, WOB, stridor and hypoxemia, drooling, moderate tongue swelling, soft palate and/or uvula swelling, continuous skin scratching (itching/pruritis) , generalised skin scratching (itching/pruritis) or excoriations, generalised skin urticaria a, skin flushing and generalised skin erythema, frequent or continuous nausea or abdominal pain and sense of being distressed due to GI symptoms.

In a further embodiment, said clinical signs and symptoms are severe clinical signs and symptoms selected from the list consisting of cardiac arrest, respiratory failure, severe swelling of the tongue, soft palate and/or uvula.

As mentioned, the treatment with a BTKi after onset of an allergic reaction might limit the progression from mild acute allergic reaction to severe allergic reaction. The administration might include one or more doses as close to the time of exposure to the allergen, or where the subject has symptoms or signs consisting with an acute allergic reaction.

Thus, in one embodiment, said subject is having symptoms or signs consisting with an acute allergic reaction or a severe allergic reaction, including anaphylaxis.

The above-mentioned signs and symptoms, ultimately leading to anaphylaxis, may not exclusively be IgE mediated. Some patients who experience anaphylaxis do not present with any evidence of IgE-dependent immune activation. In these patients, there are no signs of IgE mediated activation of mast cells or basophils as suggested by a positive skin test result, presence of allergen-specific IgE, or elevated histamine. In some patients, there is no evidence of MC activation at all; for example, they have normal tryptase (Cianferoni 2021)

Non-IgE mediated anaphylaxis can be triggered for example by the complement system or through activation of mast cells by G-protein coupled receptors, which are highly expressed on the surface of mast cells. This activation can for examples be triggered by different types of drugs, including oversulfated heparin, aspirin and other NSAIDs, antibiotics, including vancomycin and the fluoroquinilones, opiates, and drugs used in general anesthesia, particularly NMBAs.

### BTKis

Compounds of claim 1 with the ability to inhibit the kinase activity of BTK inside mast cells and/or basophils are eligible for being used in the methods and uses described herein.

Preferred are BTKis which has fast onset of systemic absorption after being administered, such as BTKi administration resulting in a C-max within 1 hour after administration, preferably within 50 minutes, such as 45, 40, 35, 30, 25, 20, 15, 12, 10, 8 or 5 minutes after administration, more preferably within 25 min, such as within 20 min, such as within 10 min.

As mentioned, a BTKi for use in embodiments described herein is a molecule capable of inhibiting the kinase activity of BTK, preferably with IC50 in the nanomolar range or lower. A BTKi may also inhibit other kinases, but with higher IC50 than for BTK inhibitory activity. The activity (IC50) may differ among the different BTKis and can be determined using a protein kinase activity assay as known to the skilled person.

**Table 1 shows BTKis that either are marketed or in development.**

| **No.** | **Name** | **Chemical name** |
|---|---|---|
| 1 | Ibrutinib | 1-[(3R)-3-[4-amino-3-(4-phenoxyphenyl)-1H-pyrazolo[3,4-d]pyrimidin-1-yl]-1-piperidinyl]-2-propen-1-one |
| 2 | Acalabrutinib | 4-{8-amino-3-[(2S)-1-(but-2-ynoyl)pyrrolidin-2-yl]imidazo[1,5-a]pyrazin-1-yl)}-N-(pyridine-2-yl)benzamide |
| 3 | Zanubrutinib | (S)-7-(1-acryloylpiperidin-4-yl)-2-(4-phenoxyphenyl)-4,5,6,7-tetrahydropyrazolo[1,5-a]pyrimidine-3-carboxamide |
| 4 | Abivertinib | N-(3-((2-((3-Fluoro-4-(4-methylpiperazin-1-yl)phenyl)amino)-7H-pyrrolo[2,3-d]pyrimidin-4-yl)oxy)phenyl)acrylamide |
| 5 | Evobrutinib | 1-(4-(((6-amino-5-(4-phenoxyphenyl)pyrimidin-4-yl)amino)methyl)piperidin-1-yl)prop-2-en-1-one |
| 6 | Tolebrutinib | 4-amino-3-(4-phenoxyphenyl)-1-[(3R)-1-prop-2-enoylpiperidin-3-yl]imidazo[4,5-c]pyridin-2-one |
| 7 | Rilzabrutinib | (E)-2-[(3R)-3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile |
| 8 | Nemtabrutinib | (2-Chloro-4-phenoxyphenyl)(4-(((3R,6S)-6-(hydroxymethyl)tetrahydro-2H-pyran-3-yl)amino)-7h-pyrrolo(2,3-d)pyrimidin-5-yl)methanone |
| 9 | Branebrutinib | 4-[(3S)-3-(but-2-ynoylamino)piperidin-1-yl]-5-fluoro-2,3-dimethyl-1H-indole-7-carboxamide |
| 10 | Elsubrutinib | (S)-4-(1-Acryloylpiperidin-3-yl)-1H-indole-7-carboxamide |
| 11 | Fenebrutinib | 10-[3-(hydroxymethyl)-4-[1-methyl-5-[[5-[(2S)-2-methyl-4-(oxetan-3-yl)piperazin-1-yl]pyridin-2-yl]amino]-6-oxopyridin-3-yl]pyridin-2-yl]-4,4-dimethyl-1,10-diazatricyclo[6.4.0.02,6]dodeca-2(6),7-dien-9-one |
| 12 | Pirtobrutinib | 5-amino-3-[4-[[(5-fluoro-2-methoxybenzoyl)amino]methyl]phenyl]-1-[(2S)-1,1,1-trifluoropropan-2-yl]pyrazole-4-carboxamide |
| 13 | Tepotinib | 3-[1-[[3-[5-[(1-methylpiperidin-4-yl)methoxy]pyrimidin-2-yl]phenyl]methyl]-6-oxopyridazin-3-yl]benzonitrile |
| 14 | Orelabrutinib | 2-(4-phenoxyphenyl)-6-(1-prop-2-enoylpiperidin-4-yl)pyridine-3-carboxamide |
| 15 | Remibrutinib | N-[3-[6-amino-5-[2-[methyl(prop-2-enoyl)amino]ethoxy]pyrimidin-4-yl]-5-fluoro-2-methylphenyl]-4-cyclopropyl-2-fluorobenzamide |
| 16 | Tirabrutinib | 6-amino-9-[(3R)-1-but-2-ynoylpyrrolidin-3-yl]-7-(4-phenoxyphenyl)purin-8-one |
| 17 | Vecabrutinib | (3R,4S)-1-(6-amino-5-fluoropyrimidin-4-yl)-3-[(3R)-3-[3-chloro-5-(trifluoromethyl)anilino]-2-oxopiperidin-1-yl]piperidine-4-carboxamide |
| 18 | BIIB068 | N-[[2-methyl-4-[2-[(1-methylpyrazol-4-yl)amino]pyrimidin-4-yl]phenyl]methyl]-3-propan-2-yloxyazetidine-1-carboxamide |
| 19 | CG-806 | 1-(3-fluoro-4-(7-(4-methyl-1H-imidazol-2-yl)-1-oxoisoindolin-4-yl)phenyl)-3-(3-(trifluoromethyl)phenyl)urea |
| 20 | Edralbrutinib | 4-amino-1-[(3R)-1-but-2-ynoylpyrrolidin-3-yl]-3-[4-(2,6-difluorophenoxy)phenyl]-6H-pyrrolo[2,3-d]pyridazin-7-one |
| 21 | Poseltinib | N-(3-((2-((4-(4-Methylpiperazin-1-yl)phenyl)amino)furo[3,2-d]pyrimidin-4-yl)oxy)phenyl)acrylamide |
| 22 | Spebrutinib | N-[3-[[5-fluoro-2-[4-(2-methoxyethoxy)anilino]pyrimidin-4-yl]amino]phenyl]prop-2-enamide |
| 23 | LFM-A13 | 2-Cyano-N-(2,5-dibromophenyl)-3-hydroxy-2-butenamide |
| 24 | (-)-Terreic acid | (1R,6S)-3-Hydroxy-4-methyl-7-oxabicyclo[4.1.0]hept-3-ene-2,5-dione |
| 25 | PCI 29732 | 1-Cyclopentyl-3-(4-phenoxyphenyl)-1H-pyrazolo[3,4-d]pyrimidin-4-amine |
| 26 | luxeptinib | 1-[3-fluoro-4-[7-(5-methyl-1H-imidazol-2-yl)-1-oxo-2,3-dihydroisoindol-4-yl]phenyl]-3-(2,4,6-trifluorophenyl)urea |
| 27 | Atuzabrutinib | (E)-2-[(3R)-3-[4-amino-3-(2-fluoro-4-phenoxyphenyl)pyrazolo[3,4-d]pyrimidin-1-yl]piperidine-1-carbonyl]-4,4-dimethylpent-2-enenitrile |
| 28 | Milrebrutinib | 2-(3-(2-amino-6-(1-(oxetan-3-yl)-1,2,3,6-tetrahydropyridin-4-yl)-7H-pyrrolo[2,3-d]pyrimidin-4-yl)-2-(hydroxymethyl)phenyl)-6-cyclopropyl-8-fluoroisoquinolin-1(2H)-one |
| 29 | BIIB-091 | 1-tert-butyl-N-[8-[2-[(1-methylpyrazol-4-yl)amino]pyrimidin-4-yl]-2-(oxetan-3-yl)-1,3,4,5-tetrahydro-2-benzazepin-5-yl]triazole-4-carboxamide |
| 30 | Olmutinib | N-[3-[2-[4-(4-methylpiperazin-1-yl)anilino]thieno[3,2-d]pyrimidin-4-yl]oxyphenyl]prop-2-enamide |
| 31 | TL-895 | 2-Propen-1-one, 1-[4-[[[6-amino-5-(4-phenoxyphenyl)-4-pyrimidinyl]amino]methyl]-4-fluoro-1-piperidinyl] |
| 32 | NX-2127 | 2-Pyrazinecarboxamide, 3-[[4-[1-[[(3S)-1-[2-(2,6-dioxo-3-piperidinyl)-2,3-dihydro-1,3-dioxo-1H-isoindol-5-yl]-3-pyrrolidinyl]methyl]-4-piperidinyl]phenyl]amino]-5-(1-piperidinyl) |
| 32 | JNJ-64264681 | 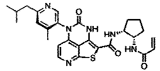 |

Further BTKis may be CT-1530, TAS-5315, WNW-1011, AC-00058, AS-0871, DTRMWXHS-12, FCN-647, GB-5121, HMPL-760, HWH-486, HZA-018, NX-2127, TAS-5315, BGB-16673, HBW-003, HSK-29116, NX-5948, SYHA-1811, YZJ-3058, ZXBT-1158, ABP-1119, ABP-1130, AC-0676, B-9, BEBT-707, BIBTK-1, DWP-213388, GB7208, HSK-26784, ING-004, or KBP-7536.

According to the present invention, the BTKi is selected from the group consisting of: 1-[(3R)-3-[4-amino-3-(4-phenoxyphenyl)-1H-pyrazolo[3,4-d]pyrimidin-1-yl]-1-piperidinyl]-2-propen-1-one (Ibrutinib), 4-{8-amino-3-[(2S)-1-(but-2-ynoyl)pyrrolidin-2-yl]imidazo[1,5-a]pyrazin-1-yl)}-N-(pyridine-2-yl)benzamide (Acalabrutinib), (S)-7-(1-acryloylpiperidin-4-yl)-2-(4-phenoxyphenyl)-4,5,6,7-tetrahydropyrazolo[1,5-a]pyrimidine-3-carboxamide (Zanubrutinib), N-(3-((2-((3-Fluoro-4-(4-methylpiperazin-1-yl)phenyl)amino)-7H-pyrrolo[2,3-d]pyrimidin-4-yl)oxy)phenyl)acrylamide (Abivertinib), 1-(4-(((6-amino-5-(4-phenoxyphenyl)pyrimidin-4-yl)amino)methyl)piperidin-1-yl)prop-2-en-1-one (Evobrutinib), 4-amino-3-(4-phenoxyphenyl)-1-[(3R)-1-prop-2-enoylpiperidin-3-yl]imidazo[4,5-c]pyridin-2-one (Tolebrutinib), (E)-2-[(3R)-3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (Rilzabrutinib), (2-Chloro-4-phenoxyphenyl)(4-(((3R,6S)-6-(hydroxymethyl)tetrahydro-2H-pyran-3-yl)amino)-7h-pyrrolo(2,3-d)pyrimidin-5-yl)methanone (Nemtabrutinib), 4-[(3S)-3-(but-2-ynoylamino)piperidin-1-yl]-5-fluoro-2,3-dimethyl-1H-indole-7-carboxamide (Branebrutinib), (S)-4-(1-Acryloylpiperidin-3-yl)-1H-indole-7-carboxamide (Elsubrutinib), 10-[3-(hydroxymethyl)-4-[1-methyl-5-[[5-[(2S)-2-methyl-4-(oxetan-3-yl)piperazin-1-yl]pyridin-2-yl]amino]-6-oxopyridin-3-yl]pyridin-2-yl]-4,4-dimethyl-1,10-diazatricyclo[6.4.0.02,6]dodeca-2(6),7-dien-9-one (Fenebrutinib), 5-amino-3-[4-[[(5-fluoro-2-methoxybenzoyl)amino]methyl]phenyl]-1-[(2S)-1,1,1-trifluoropropan-2-yl]pyrazole-4-carboxamide (Pirtobrutinib), 3-[1-[[3-[5-[(1-methylpiperidin-4-yl)methoxy]pyrimidin-2-yl]phenyl]methyl]-6-oxopyridazin-3-yl]benzonitrile (Tepotinib), 2-(4-phenoxyphenyl)-6-(1-prop-2-enoylpiperidin-4-yl)pyridine-3-carboxamide (Orelabrutinib), N-[3-[6-amino-5-[2-[methyl(prop-2-enoyl)amino]ethoxy]pyrimidin-4-yl]-5-fluoro-2-methylphenyl]-4-cyclopropyl-2-fluorobenzamide (Remibrutinib), 6-amino-9-[(3R)-1-but-2-ynoylpyrrolidin-3-yl]-7-(4-phenoxyphenyl)purin-8-one (Tirabrutinib), (3R,4S)-1-(6-amino-5-fluoropyrimidin-4-yl)-3-[(3R)-3-[3-chloro-5-(trifluoromethyl)anilino]-2-oxopiperidin-1-yl]piperidine-4-carboxamide (Vecabrutinib), N-[[2-methyl-4-[2-[(1-methylpyrazol-4-yl)amino]pyrimidin-4-yl]phenyl]methyl]-3-propan-2-yloxyazetidine-1-carboxamide (BiiB068), 1-(3-fluoro-4-(7-(4-methyl-1H-imidazol-2-yl)-1-oxoisoindolin-4-yl) phenyl)-3-(3-(trifluoromethyl)phenyl)urea (CG-806), 4-amino-1-[(3R)-1-but-2-ynoylpyrrolidin-3-yl]-3-[4-(2,6-difluorophenoxy)phenyl]-6H-pyrrolo[2,3-d]pyridazin-7-one (Edralbrutinib), N-(3-((2-((4-(4-Methylpiperazin-1-yl)phenyl)amino)furo[3,2-d]pyrimidin-4-yl)oxy)phenyl)acrylamide (Poseltinib), N-[3-[[5-fluoro-2-[4-(2-methoxyethoxy)anilino]pyrimidin-4-yl]amino]phenyl]prop-2-enamide (Spebrutinib), 2-Cyano-N-(2,5-dibromophenyl)-3-hydroxy-2-butenamide (LFM-A13), (1R,6S)-3-Hydroxy-4-methyl-7-oxabicyclo[4.1.0]hept-3-ene-2,5-dione ((-)-Terreic acid), 1-Cyclopentyl-3-(4-phenoxyphenyl)-1H-pyrazolo[3,4-d]pyrimidin-4-amine (PCI 29732), 1-[3-fluoro-4-[7-(5-methyl-1H-imidazol-2-yl)-1-oxo-2,3-dihydroisoindol-4-yl]phenyl]-3-(2,4,6-trifluorophenyl)urea (luxeptinib), (E)-2-[(3R)-3-[4-amino-3-(2-fluoro-4-phenoxyphenyl)pyrazolo[3,4-d]pyrimidin-1-yl]piperidine-1-carbonyl]-4,4-dimethylpent-2-enenitrile (Atuzabrutinib), 2-(3-(2-amino-6-(1-(oxetan-3-yl)-1,2,3,6-tetrahydropyridin-4-yl)-7H-pyrrolo[2,3-d]pyrimidin-4-yl)-2-(hydroxymethyl)phenyl)-6-cyclopropyl-8-fluoroisoquinolin-1(2H)-one (Milrebrutinib), 1-tert-butyl-N-[8-[2-[(1-methylpyrazol-4-yl)amino]pyrimidin-4-yl]-2-(oxetan-3-yl)-1,3,4,5-tetrahydro-2-benzazepin-5-yl]triazole-4-carboxamide (BIIB-091), N-[3-[2-[4-(4-methylpiperazin-1-yl)anilino]thieno[3,2-d]pyrimidin-4-yl]oxyphenyl]prop-2-enamide (Olmutinib), 2-Propen-1-one, 1-[4-[[[6-amino-5-(4-phenoxyphenyl)-4-pyrimidinyl]amino]methyl]-4-fluoro-1-piperidinyl] (TL-895), and 2-Pyrazinecarboxamide, 3-[[4-[1-[[(3S)-1-[2-(2,6-dioxo-3-piperidinyl)-2,3-dihydro-1,3-dioxo-1H-isoindol-5-yl]-3-pyrrolidinyl]methyl]-4-piperidinyl]phenyl]amino]-5-(1-piperidinyl) (NX-2127).

In a preferred embodiment, the BTKi is selected from the group consisting of 1-[(3R)-3-[4-amino-3-(4-phenoxyphenyl)-1H-pyrazolo[3,4-d]pyrimidin-1-yl]-1-piperidinyl]-2-propen-1-one (Ibrutinib), 4-{8-amino-3-[(2S)-1-(but-2-ynoyl)pyrrolidin-2-yl]imidazo[1,5-a]pyrazin-1-yl)}-N-(pyridine-2-yl)benzamide (Acalabrutinib), (S)-7-(1-acryloylpiperidin-4-yl)-2-(4-phenoxyphenyl)-4,5,6,7-tetrahydropyrazolo[1,5-a]pyrimidine-3-carboxamide (Zanubrutinib).

In a more preferred embodiment, the BTKi is 4-{8-amino-3-[(2S)-1-(but-2-ynoyl)pyrrolidin-2-yl]imidazo[1,5-a]pyrazin-1-yl)}-N-(pyridine-½2-yl)benzamide (Acalabrutinib).

In another preferred embodiment, the BTKi is 4-[(3S)-3-(but-2-ynoylamino)piperidin-1-yl]-5-fluoro-2,3-dimethyl-1H-indole-7-carboxamide (Branebrutinib) or N-[3-[6-amino-5-[2-[methyl(prop-2-enoyl)amino]ethoxy]pyrimidin-4-yl]-5-fluoro-2-methylphenyl]-4-cyclopropyl-2-fluorobenzamide (Remibrutinib), preferably N-[3-[6-amino-5-[2-[methyl(prop-2-enoyl)amino]ethoxy]pyrimidin-4-yl]-5-fluoro-2-methylphenyl]-4-cyclopropyl-2-fluorobenzamide (Remibrutinib).

It should be understood that in connection with treating a severe allergic reaction, such as anaphylaxis, the administration of the BTKi might reduce the need of epinephrine treatment and/or second-line treatment with antihistamines or steroids.

Epinephrine treatment is today the first line of treatment if a human is experiencing a severe allergic reaction including anaphylaxis, whereas antihistamines and glucocorticoids are considered solely second-line therapy. Antihistamines, having a slow onset of action, are unable to stabilize or prevent mast cell degranulation, and unable to target other mediators of the severe allergic reaction than histamine. Further, antihistamines will not effectively treat cardiovascular and respiratory symptoms such as hypotension or bronchospasm.

Therefore, there is an unmet need for alternative options or adjunctive options for first-line treatment as well as second-line treatment of severe allergic reactions, including anaphylaxis.

Therefore, in one embodiment, the BTKi might be administered before and/or after and/or concurrently with epinephrine treatment. Thus, the BTKi might be used as a combination therapy with epinephrine. Preferably, the BTKi may be administered before the epinephrine treatment.

In another embodiment, administration of the BTKi reduces the need of epinephrine treatment and/or second-line treatment with antihistamine or steroid.

In a further or alternative embodiment, the BTKi may be administered as a second-line drug.

Thus, the administration of the BTKi may replace i.v. antihistamine or it may be administered in combination with antihistamine treatment.

### Mode of administration and doses

Administration of a BTKi can be carried out in several ways as described in the following, non-limiting examples.

The BTKi or more specifically a composition, such as a dosage form, comprising the BTKi might preferably be administered by any route resulting in a fast presence / uptake of the BTKi into the blood in therapeutically relevant doses. Conventionally, a BTKi might be administered by peroral administration, typically as a tablet. However, the bioavailability following peroral administration is generally low and peroral administration might not always be preferred when a fast uptake of the BTKi is desirable. Therefore, other routes of administration are preferable, such as parenteral routes including mucosal administration routes. By parenteral administration, the BTKi is delivered directly into the blood stream without having to pass the oral or alimentary mucous layer. Mucosal administration may typically include buccal, sublingual, nasal, ocular, rectal, or pulmonal administration, such as preferably buccal, sublingual or intranasal administration. Accordingly, in one embodiment of the present invention, the BTKi or the dosage form comprising the BTKi is administered by mucosal administration, such as preferably by oromucosal administration, such as preferably by administration to the buccal, sublingual and/or pharynx mucosa. Oromucosal administration comprises any administration method, wherein the formulation in part or in fully meets the mucosa of the oral cavity and/or the pharynx of the patient. Oromucosal administration methods include sublingual administration and buccal administration. In a preferred embodiment, the mucosal administration is sublingual administration.

Further the BTKi, or a composition comprising the BTKi, can be administered by intradermal injection, which is a delivery of the vaccine into the dermis of the skin, located between epidermis and the hypodermis. Alternatively, the vaccine can be administered intravenously, which is an administration directly into the blood stream of the subject. Further, administration of the vaccine intramuscular is an injection into the muscles of the subject. In addition, the vaccine can be administered subcutaneous, which is under the skin, in the area between the muscle and the skin of the subject. Transdermal administration includes administration across the skin.

As mentioned, any mode of administration can be used as long as the mode results in the presence of a dose of the BTKi in the desired target (e.g., blood basophils or mast cells in the tissues), in an amount sufficient to generate the desired response in a human in need of such response. Overall, it is considered that the BTKi may be administered by injection (e.g., intravenous, intramuscular or subcutaneous injection, preferably by intramuscular injection), by transmucosal delivery/administration (e.g., sublingually, intranasally or by inhalation). In the alternative, the BTKi might be administered perorally.

While current marketed products of BTKi is in a peroral dosage form (tablet, capsule or suspension), the BTKi may be eligible for being formulated into liquid formulations for injection or into non-parenteral dosage forms, like nasal administrable forms, which can be either liquid or powder sprays. Non-parenteral dosage forms would be preferred over the peroral dosage form for BTKis having low peroral bioavailability and high first pass metabolism. If peroral administration is preferred, the BTKi may be formulated as a suspension, such as a suspension that provides faster uptake and Tmax of the BTKi compared to a capsule or a tablet formulation of the same BTKi.

As mentioned, the BTKi should be administered shortly after exposure to an offending allergen or following the initial signs or symptoms of an allergic reaction triggered by the allergen. Typically, the dose required might be a single dose, eventually a repeated administration at a later point in time might take place, for example if delayed allergic reactions occur or the first dose does not sufficiently reduce the clinical signs or symptoms. Therefore, depending on the individual case, the administration of the BTKi can be repeated following the initial dose.

In one embodiment of the present invention, the BTKi is administered as a single dose, optionally wherein one or more doses of the BTKi is administered following the first dose within 24-hours from the first dose.

A peroral dose is typically within the range of 50 mg to 500 mg, such as 100 to 200 mg, however dependent on the BTKi. The dose is expected to be much lower with non-parenteral administration and still reach similar blood concentrations to peroral administration.

In one embodiment of the present invention, the administration of the BTKi results in a C-max within 1 hour after administration, preferably within 50 minutes, such as 45, 40, 35, 30, 25, 20, 15, 12, 10, 8 or 5 minutes after administration.

In the examples, administration of a BTK inhibitor before allergen challenge is included solely as reference data for comparison, whereas only the post-exposure administration reflects the invention as claimed. EXAMPLE 1 *Inhibition of basophil activation using BTKi.*

### Aim

The aim of the present study was to test the effect of a BTKi added to basophils after activation by exposure to an allergen or by non-IgE mediated activators.

### Materials and Methods

### Cells

PBMCs were isolated from heparinized blood using Leucoseptubes (Greiner 227390) with Lymfoprep (Fresenius Kabi 1114547). Following isolation, the cells were washed and resuspended in RPMI 0.5% HSA (RPMI 1640 Gibco 72400-021, HSA Sigma A8763) to 0.25x original blood volume. 2 ng/ml of cytokine IL-3 (RD Peprotec cat nr 200-03) was added to the cells for Basophil priming with final 1 ng/ml. 50 µl of the cell suspension was used for each test, corresponding to 200 µl blood.

### BTK inhibitor:

The test can be carried out with any BTKi. In the present example, the following BTKis were tested:
- Acalabrutinib (Cat A-7337, 100 mM in DMSO) was diluted in RPMI 0,5%HSA to a concentration of 10,000 and 2,000 nM.
- Ibrutinib was diluted in RPMI 0.5% HSA to a concentration of 20.000, 5.000 and 1.000 nM.
- Branebrutinib was diluted in RPMI 0.5% HSA to a concentration of 20.000 and 5.000 nM.
- Remibrutinib was diluted in RPMI 0.5% HSA to a concentration of 20.000 and 5.000 nM.
- Pirtobrutinib was diluted in RPMI 0.5% HSA to a concentration of 20.000 and 5.000 nM.

### Activation stimuli

Allergens: Grass pollen extract of the species *Phleum pratense* (ALK) was diluted in RPMI 0,5%-HSA to five concentrations of 100, 10, 1.0, 0.1 and 0.01 ng/ml. House dust mite allergen extract of the species *Dermatophagoides pterinussinus* (Der*p* ex) were made in concentrations of 0.1, 1 and 10ug/ml by dilution in RPMI 0.5% HSA.

Non-IgE mediated activators: including Con A (in concentration of 2 and 20ug/ml), and as a control a-IgE (in concentration of 10, 100 and 1000 ug/ml)
Anti-human IgE (Dako A0094) was diluted in RPMI 0.5% HSA to two activation concentrations of 1,000 and 100 ng/ml.

### Procedure:

The experiment was performed in 96 deep-well plates. 50 µl cell suspension (as described above) was added to each well. 50 µl activation stimuli and 100 µl BTK inhibitor or buffer were added at different time points (t=0 min, 5 min, 15 min or 30 min). Following addition of the components, the plate was incubated at 37°C water bath for one hour. After incubation, the activation was stopped by adding 500 µl FACS FLOW (BD342003), 0.5% BSA (Fisher scientific BP9703-100) +10mM EDTA (Invitrogen 15575-038). The plate was centrifuged for 5 min, at 600xG and 4°C. Supernatants were harvested and used for measurement of histamine by ELISA (supplier). Cell pellets were further washed and stained for FACS analysis with CD63 FITC (BD 557288) CD203c APC (e-bio) and CD123 PE (BD340545) using standard FACS staining procedure, 30 min on ice protected from light, followed by fixation with Cell Fix (BD) before analysis on Fortessa flowcytometer. Flowcytometer data was analyzed using FlowJo software and statistical data from here used for data analysis.

### Results

Cell activation and Histamine release were measured after allergen stimulation either without BTKi exposure or with BTKi exposure before allergen stimulation (data not shown) or at 1, 5, 15 and 30 min after allergen stimulation. Cells stimulated with allergen only, were activated in a dose dependent manner (figures 1A, C, E and figures 2-5, black dots). When the BTKi was added to the cell suspension, both before (data not shown) and after allergen stimulation, a dose dependent decrease in the activation was observed (figures 1A, C, E and figures 2-5 grey squares, and triangles). In addition, histamine release after acalabrutinib stimulation was measured by ELISA as previously described. Like cell activation, histamine release was strongly inhibited in the cells exposed to acalabrutinib in a dose dependent manner, compared to the cells only exposed to increasing concentration of allergen (figures 1B, D, F). Each figure (figures 1, 2 and 5) represents a single individual donor, whereas figures 3 and 4 represents 3 and 2 individual donors, respectively. In total, five donors have been analyzed, showing same results.

Further, cell activation was also measured after stimulation with non-IgE mediated activators, either without BTKi exposure, BTKi administered 30 min before stimulation or BTKi administered 5 minutes post stimulation as described above. a-IgE, Derp ex (both serves as control) (figure 8a, c, e and g) and Con A (figure 8b, d, f and h) were all able to activate the cells. Further by exposing the cells to acalabrutinib, the cell activation was inhibited in a dose dependent manner. This was seen when acalabrutinib was administered at 30 min before (figure 8a-b and e-f) and 5 min after activation (figure 8c-d and g-h).

### Conclusion

In conclusion, the present example shows that the BTKis Acalabrutinib, Ibrutinib, Pirtobrutinib, Remibrutinib, and Branebrutinib all were able to decrease the activation of cells after stimulation with an allergen. All BTKis were able to decrease the activation in a dose dependent manner, when administered both before and after allergen stimulation. Further, acalabrutinib was able to decrease the activation in a dose dependent manner, when administered both before and after non-IgE mediated stimulation of the cells. the present example is not limited to the five BTKis used. Any BTKi can be tested in this setup.

### EXAMPLE 2

*In vivo test of BTKis ability to stop an ongoing allergic reaction.*

### Aim

The aim of the present example is to evaluate the ability of the BTK inhibitor acalabrutinib to disrupt anaphylaxis *in vivo.*

### Materials and methods

C57BL/6 mice (RRID:IMSR_JAX:000664) were obtained from The Jackson Laboratory and housed in our facility for at least one week before start of intragastric sensitisations with 200µl peanut extract (1mg protein per mouse, ALK internal) and cholera toxin (5µg/mouse, List Labs, #110B). Mice received four sensitisation doses, one per week, before intraperitoneal (i.p.) challenge with 200µl peanut extract (100µg protein per mouse) nine days after the last sensitisation. B6.Cg-Fcer1atm1Knt Tg(FCER1A)1Bhk/J mice (RRID:IMSR_JAX:010506) were originally obtained from The Jackson Laboratory and then bred in house for use in a passive systemic anaphylaxis (PSA) model. Mice were sensitized by i.p. injection of a mixture of three Der p 2-specific recombinant human IgE clones: H10, H12, P4E (Christensen et al. 2008), 200µl per mouse with 16.67µg/ml of each Ab in PBS. 24 hours later, the mice were challenged by i.p. injection with 200µl allergen (0,05µg/ml rDer p 2, ALK internal). In either experiment setup, mice were gavaged with 200µl acidified water (pH 3) or 8mM acalabrutinib (MedChemExpress, HY-17600) in acidified water four hours or five minutes pre-challenge, or five minutes post-challenge. Core body temperature was measured using temperature transponders (IPTT-300, implanted subcutaneously at least a day before experiment start), directly before allergen challenge and every 5 minutes thereafter.

### Results

In the active sensitisation model of mice sensitised and challenged with peanut extract, acalabrutinib administered by intragastric gavage before allergen challenge can attenuate anaphylaxis severity significantly, as expected. Importantly, administration five minutes post-challenge significantly reduced the severity of the anaphylactic reaction as measured by decrease in body temperature.

The results of the passive sensitisation model mirror that of the active sensitisation model, BTKi treatment before challenge significantly reduces anaphylaxis severity. Treatment after challenge still has an effect but did not reach significance in the one experiment performed.

### Conclusion

The BTKi acalabrutinib given after allergen challenge is able to attenuate anaphylaxis severity in vivo both in a murine active allergic sensitisation model as well as a passive systemic anaphylaxis model.

### EXAMPLE 3

*In vivo test of BTKis ability to stop an ongoing allergic reaction in human adults.*

### Aim

This example describes a human trial outline to evaluate whether mast cell degranulation can be reduced by administering a single dose of BTKi close in time to the exposure of an agent (triggering agent) causing mast cell degranulation. It is then expected that BTKi may be a treatment concept for acute severe allergic reactions, such as anaphylaxis, with the option to administer the BTKi just before a person is in risk of being exposed to the "triggering agent" or even more advantageously to administer the BTKi just after being exposed to the triggering agent, but preferably prior to or when the person becomes aware of the initial symptoms of a severe allergic reaction after that exposure.

### Trial outline

The effect on mast cell degranulation will be investigated by the effect of single dose BTKi on the skin wheal size developing after an allergic person is being tested by skin prick testing (SPT) with an allergen the allergic person is sensitized to. The effect on the allergic reaction will be determined by comparing the wheal size developed before and after intake of the BTKi and with the consideration that it takes time to develop the wheal. Therefore, the wheal size will be assessed about 15 minutes after the start of the allergen challenge.

In the following, one way of performing the skin prick testing and an evaluation of BTKi impact on wheal size development are described. The test can be carried out by other allergens and trial medication and skin prick testing procedure as known in the art.

### Material and methods

**Trial subjects:** Adults with a clinical history of an IgE mediated allergic reaction towards grass pollen, serum IgE to grass pollen ≥ 0.70 kUA/L and SPT to grass pollen ≥ 5 mm.

**Trial medication:** Calquence^{®} capsules 100 mg (Active ingredient is acalubrutinib). Trial subjects will be treated with a single dose of oral suspension of 200 mg acalabrutinib (two capsules suspended in a solution of degassed Coca-Cola^{®} as described in Sharma et al. (2022). Subjects will be fasting for six hours before dosing of acalabrutinib.

**Skin prick testing:** Skin prick testing will be performed at a screening visit, at a treatment visit and at a follow up visit 7 days after the treatment. At the treatment visit, skin prick testing will be performed 15 and 5 minutes before intake of acalabrutinib and at 0, 5, 10, 15, 30 and 60 minutes after intake of acalabrutinib.

Skin prick testing will at all time points include duplicate testing with grass pollen allergen extract (Soluprick^{®} SQ Grass Pollen Phleum pratense (Timothy Grass) 10 HEP from ALK-Abelló) and a single positive control with histamine (Soluprick^{®} positive control with 10 mg/ml histamine from ALK-Abelló). Skin prick testing during the screening visit, 15 minutes prior to intake of acalabrutinib and at follow up will also include a negative control without grass pollen allergen extract (Soluprick^{®} negative control with no allergen content).

The wheal size of the skin prick tests will be compared to the wheal size (mm) of the skin prick test performed 15 minutes before intake of acalabrutinib by the absolute and the relative differences in skin prick test wheal size.

**Pharmacokinetics:** Blood samples will be collected at different time points (0, 5, 10, 15, 30 and 60 minutes) after intake of trial medication to determine the blood concentration of acalabrutinib and optionally its active metabolite.

**Adverse event monitoring:** Relevant vital signs, physical examination, electrocardiogram (ECG), and clinical laboratory values during treatment and follow up visit

### Assessments:

- The change in skin prick test wheal size between the skin prick test at each time point (at 5 minutes before intake of acalabrutinib and at 0, 5, 10, 15, 30 and 60 minutes after intake of acalabrutinib) and the skin prick test performed 15 minutes prior to intake of acalabrutinib.
- Bioavailability data such as Tmax, Cmax, earliest detectable concentration of acalabrutinib and/or active metabolite in the blood.
- Safety, adverse events (AEs) and tolerability are evaluated.

Overview of assessments to be performed during the trial:

| **Assessments** | **Timing (minutes) in the assessments before and after intake of trial medication** | | | | | | | | | |
|---|---|---|---|---|---|---|---|---|---|---|
| | Before **-** 15 | -15 min | -5 min | 0 | +5 min | +10 min | +15 min | +30 min | +60 min | After 60 min |
| Physical examination | X | | | | | | | | | X |
| SPT grass and positive control | | X | X | X | X | X | X | X | X | |
| SPT negative control | | X | | | | | | | | |
| ECG | X | | | | | | | | | X |
| Blood collection | | | | X | X | X | X | X | X | |
| Assess and record AEs in eCRF | X | X | X | X | X | X | X | X | X | X |
| Record need of concomitant medication | X | X | X | X | X | X | X | X | X | X |
| Intake of trial medication | | | | X | | | | | | |

### LIST OF REFERENCES

Adriana et al. (2022) Anaphylaxis: Definition, Epidemiology, Diagnostic challenges, Grading system. Immunology and Allergy Clinics. 42:1-11.

Bagos-Estevez AG, Ledford DK (2022) Anaphylaxis. Immunology and Allergy Clinics of North America 42:1-11

Brullo C, Villa C, Tasso B, et al. (2021) Btk Inhibitors: A Medicinal Chemistry and Drug Delivery Perspective. International Journal of Molecular Sciences 22.

Cianferoni A (2021) Non-IgE mediated anaphylaxis. Clinicacal reviews in allergy and immunology

Dispenza, Pongracic, Singh, Bochner (2018) Short-term ibrutinib therapy suppresses skin test responses and eliminates IgE-mediated basophil activation in adults with peanut or tree nut allergy. Journal of Allergy and Clinical Immunology, The 141:1914-1916.e7.

Dispenza MC, Krier-Burris RA, Chhiba KD, et al (2020) Bruton's tyrosine kinase inhibition effectively protects against human IgE-mediated anaphylaxis. Journal of Clinical Investigation 130:4759-4770.

Dispenza MC (2021) The Use of Bruton's Tyrosine Kinase Inhibitors to Treat Allergic Disorders. Current Treatment Options in Allergy 8:261-273.

Dribin, Schnadower, Spergel, et al. (2021) Severity grading system for acute allergic reactions: A multidisciplinary Delphi study. Journal of Allergy and Clinical Immunology, 148:173-181.

Ellmeier W, Abramova A, Schebesta A (2011) Tec family kinases: regulation of FcεRI-mediated mast-cell activation. FEBS Journal, 278:1990-2000.

Estupiñán HY, Berglöf A, Zain R, Smith CIE (2021) Comparative Analysis of BTK Inhibitors and Mechanisms Underlying Adverse Effects. Frontiers in Cell and Developmental Biology 9:630942.

Lipsky A, Lamanna N. Managing toxicities of Bruton's tyrosine kinase inhibitors. Hematology / the Education Program of the American Society of Hematology Publisher: American Society of Hematology, Vol 2020 Issue 1, pp 336-345, 2020.

Sampson HA, Munoz-Furlong A, Campbell RL, et al. (2006) Second Symposium on the Definition and Management of Anaphylaxis: Summary Report-Second National Institute of Allergy and Infectious Disease/Food Allergy and Anaphylaxis Network Symposium. Annals of Emergency Medicine 47:373-380.

Sharma et al. Bioavailability of acalabrutinib suspension delivered via nasogastric tube in the presence or absence of a proton pump inhibitor in healthy subjects. British Journal of Clinical Pharmacology, vol 88, pp 4573-4584, 2022.

Smiljkovic D, Blatt K, Stefanzl G, et al. (2017) BTK inhibition is a potent approach to block IgE-mediated histamine release in human basophils. Allergy 72:1666-1676.

Wen T, Wang J, Shi Y, et al. (2021) Inhibitors targeting Bruton's tyrosine kinase in cancers: drug development advances. Leukemia 35:312-332.

Zhang H and Qiu L (2019) Brutons Tyrosine Kinase (BTK) inhibitors as sensitizing Agents for Cancer Chemotherapy. Cancer sensitizing agents for chemotherapy 4:109-124.

## Claims

1. A BTKi for use in a method of treating an allergic reaction in a subject in need thereof, wherein the method comprises administering a therapeutically effective dose of a Bruton's tyrosine kinase inhibitor (BTKi) to said subject, wherein the BTKi is administered within a period ranging from the subject's exposure to an agent triggering said allergic reaction until 24 hours after said exposure,
wherein the BTKi is selected from the list consisting of 1-[(3R)-3-[4-amino-3-(4-phenoxyphenyl)-1H-pyrazolo[3,4-d]pyrimidin-1-yl]-1-piperidinyl]-2-propen-1-one (Ibrutinib), 4-{8-amino-3-[(2S)-1-(but-2-ynoyl)pyrrolidin-2-yl]imidazo[1,5-a]pyrazin-1-yl)}-N-(pyridine-2-yl)benzamide (Acalabrutinib), (S)-7-(1-acryloylpiperidin-4-yl)-2-(4-phenoxyphenyl)-4,5,6,7-tetrahydropyrazolo[1,5-a]pyrimidine-3-carboxamide (Zanubrutinib), N-(3-((2-((3-Fluoro-4-(4-methylpiperazin-1-yl)phenyl)amino)-7H-pyrrolo[2,3-d]pyrimidin-4-yl)oxy)phenyl)acrylamide (Abivertinib), 1-(4-(((6-amino-5-(4-phenoxyphenyl)pyrimidin-4-yl)amino)methyl)piperidin-1-yl)prop-2-en-1-one (Evobrutinib), 4-amino-3-(4-phenoxyphenyl)-1-[(3R)-1-prop-2-enoylpiperidin-3-yl]limidazo[4,5-c]pyridin-2-one (Tolebrutinib), (E)-2-[(3R)-3-[4-amino-3-(2-fluoro-4-phenoxyphenyl)pyrazolo[3,4-d]pyrimidin-1-yl]piperidine-1-carbonyl]-4-methyl-4-[4-(oxetan-3-yl)piperazin-1-yl]pent-2-enenitrile (Rilzabrutinib), (2-Chloro-4-phenoxyphenyl)(4-(((3R,6S)-6-(hydroxymethyl)tetrahydro-2H-pyran-3-yl)amino)-7h-pyrrolo(2,3-d)pyrimidin-5-yl)methanone (Nemtabrutinib), 4-[(3S)-3-(but-2-ynoylamino)piperidin-1-yl]-5-fluoro-2,3-dimethyl-1H-indole-7-carboxamide (Branebrutinib), (S)-4-(1-Acryloylpiperidin-3-yl)-1H-indole-7-carboxamide (Elsubrutinib), 10-[3-(hydroxymethyl)-4-[1-methyl-5-[[5-[(2S)-2-methyl-4-(oxetan-3-yl)piperazin-1-yl]pyridin-2-yl]amino]-6-oxopyridin-3-yl]pyridin-2-yl]-4,4-dimethyl-1,10-diazatricyclo[6.4.0.02,6]dodeca-2(6),7-dien-9-one (Fenebrutinib), 5-amino-3-[4-[[(5-fluoro-2-methoxybenzoyl)amino]methyl]phenyl]-1-[(2S)-1,1,1-trifluoropropan-2-yl]pyrazole-4-carboxamide (Pirtobrutinib), 3-[1-[[3-[5-[(1-methylpiperidin-4-yl)methoxy]pyrimidin-2-yl]phenyl]methyl]-6-oxopyridazin-3-yl]benzonitrile (Tepotinib), 2-(4-phenoxyphenyl)-6-(1-prop-2-enoylpiperidin-4-yl)pyridine-3-carboxamide (Orelabrutinib), N-[3-[6-amino-5-[2-[methyl(prop-2-enoyl)amino]ethoxy]pyrimidin-4-yl]-5-fluoro-2-methylphenyl]-4-cyclopropyl-2-fluorobenzamide (Remibrutinib), 6-amino-9-[(3R)-1-but-2-ynoylpyrrolidin-3-yl]-7-(4-phenoxyphenyl)purin-8-one (Tirabrutinib), (3R,4S)-1-(6-amino-5-fluoropyrimidin-4-yl)-3-[(3R)-3-[3-chloro-5-(trifluoromethyl)anilino]-2-oxopiperidin-1-yl]piperidine-4-carboxamide (Vecabrutinib), N-[[2-methyl-4-[2-[(1-methylpyrazol-4-yl)amino]pyrimidin-4-yl]phenyl]methyl]-3-propan-2-yloxyazetidine-1-carboxamide (BiiB068), 1-(3-fluoro-4-(7-(4-methyl-1H-imidazol-2-yl)-1-oxoisoindolin-4-yl)phenyl)-3-(3-(trifluoromethyl)phenyl)urea (CG-806), 4-amino-1-[(3R)-1-but-2-ynoylpyrrolidin-3-yl]-3-[4-(2,6-difluorophenoxy)phenyl]-6H-pyrrolo[2,3-d]pyridazin-7-one (Edralbrutinib), N-(3-((2-((4-(4-Methylpiperazin-1-yl)phenyl)amino)furo[3,2-d]pyrimidin-4-yl)oxy)phenyl)acrylamide (Poseltinib), N-[3-[[5-fluoro-2-[4-(2-methoxyethoxy)anilino]pyrimidin-4-yl]amino]phenyl]prop-2-enamide (Spebrutinib), 2-Cyano-N-(2,5-dibromophenyl)-3-hydroxy-2-butenamide (LFM-A13), (1R,6S)-3-Hydroxy-4-methyl-7-oxabicyclo[4.1.0]hept-3-ene-2,5-dione ((-)-Terreic acid), 1-Cyclopentyl-3-(4-phenoxyphenyl)-1H-pyrazolo[3,4-d]pyrimidin-4-amine (PCI 29732), 1-[3-fluoro-4-[7-(5-methyl-1H-imidazol-2-yl)-1-oxo-2,3-dihydroisoindol-4-yl]phenyl]-3-(2,4,6-trifluorophenyl)urea (luxeptinib), (E)-2-[(3R)-3-[4-amino-3-(2-fluoro-4-phenoxyphenyl)pyrazolo[3,4-d]pyrimidin-1-yl]piperidine-1-carbonyl]-4,4-dimethylpent-2-enenitrile (Atuzabrutinib), 2-(3-(2-amino-6-(1-(oxetan-3-yl)-1,2,3,6-tetrahydropyridin-4-yl)-7H-pyrrolo[2,3-d]pyrimidin-4-yl)-2-(hydroxymethyl)phenyl)-6-cyclopropyl-8-fluoroisoquinolin-1(2H)-one (Milrebrutinib), 1-tert-butyl-N-[8-[2-[(1-methylpyrazol-4-yl)amino]pyrimidin-4-yl]-2-(oxetan-3-yl)-1,3,4,5-tetrahydro-2-benzazepin-5-yl]triazole-4-carboxamide (BIIB-091), N-[3-[2-[4-(4-methylpiperazin-1-yl)anilino]thieno[3,2-d]pyrimidin-4-yl]oxyphenyl]prop-2-enamide (Olmutinib), 2-Propen-1-one, 1-[4-[[[6-amino-5-(4-phenoxyphenyl)-4-pyrimidinyl]amino]methyl]-4-fluoro-1-piperidinyl] (TL-895), and 2-Pyrazinecarboxamide, 3-[[4-[1-[[(3S)-1-[2-(2,6-dioxo-3-piperidinyl)-2,3-dihydro-1,3-dioxo-1H-isoindol-5-yl]-3-pyrrolidinyl]methyl]-4-piperidinyl]phenyl]amino]-5-(1-piperidinyl) (NX-2127).

2. The BTKi for use in a method according to claim 1, wherein the BTKi is administered within a period ranging from the subject's exposure to the agent triggering said allergic reaction until 2 hours after said exposure.

3. The BTKi for use in a method according to claim 1 or claim 2, wherein the BTKi is administered within a period ranging from the subject's exposure to the agent triggering said allergic reaction until 30 min after said exposure.

4. The BTKi for use in a method according to anyone of the preceding claims, wherein the BTKi is administered to said subject in need thereof prior to or when the subject in need thereof has experienced a first clinical sign or symptom of an allergic response developed in consequence to said exposure.

5. The BTKi for use in a method according to anyone of the preceding claims, wherein said BTKi is reducing degranulation of mast cells and/or basophils in said subject in need thereof, which degranulation is triggered by exposure to said agent able to trigger said allergic reaction.

6. The BTKi for use in a method according to anyone of claims 1-5, wherein the method for treating the allergic reaction comprises preventing an ongoing allergic reaction to progress into a severe allergic reaction or anaphylaxis.

7. The BTKi for use in a method according to anyone of claims 1-5, wherein the method for treating the allergic reaction comprises emergency treatment of a subject who has been accidentally exposed to an agent causing or triggering said allergic reaction.

8. The BTKi for use in a method according to anyone of claims 1-5, wherein the method for treating the allergic reaction comprises reducing the subjects need for epinephrine administration and/or second-line treatment with antihistamine and/or corticosteroids.

9. The BTKi for use in a method according to anyone of the preceding claims, wherein said subject is accidentally exposed to said agent.

10. The BTKi for use in a method according to anyone of the preceding claims, wherein the BTKi is administered as a single dose, optionally wherein one or more doses are administered following the first dose within 24 hours from the first dose.

11. The BTKi for use in a method according to anyone of the preceding claims, wherein the agent is an allergen, preferably, a pollen allergen, a mite allergen, a food allergen, an insect venom allergen or a medicine.

12. The BTKi for use in a method according to claims 1-10, wherein the agent able to trigger said allergic reaction is an agent able to trigger non-IgE mediated activation of mast cells and/or basophils.

13. The BTKi for use in a method according to anyone of the preceding claims, wherein the BTKi is administered as a combination therapy with epinephrine.

14. The BTKi for use in a method according to anyone of the preceding claims, wherein the BTKi is administered as a second-line drug as a replacement for i.v. antihistamine or corticosteroid or is administered as adjunctive treatment with antihistamine or corticosteroid.

15. The BTKi for use in a method according to anyone of the preceding claims, wherein said method of treating an allergic reaction is a method of treating a delayed allergic reaction or a combined acute and delayed allergic reaction, wherein the delayed allergic reaction occurs between 2-48 hours after the allergic subject has been exposed to the agent triggering or causing the allergic reaction.

## Patentansprüche

1. BTKi zur Verwendung bei einem Verfahren des Behandelns einer allergischen Reaktion bei einem Subjekt, die dessen bedarf, wobei das Verfahren Verabreichen, dem Subjekt, einer therapeutisch wirksamen Dosis eines Bruton-Tyrosinkinase-Inhibitors (BTKi) umfasst, wobei der BTKi innerhalb einer Zeitspanne, die vom Aussetzen des Subjekts einem Agens, das die allergische Reaktion auslöst, bis 24 Stunden nach dem Aussetzen reicht, verabreicht wird,
wobei der BTKi aus der Liste ausgewählt wird bestehend aus 1-[(3R)-3-[4-Amino-3-(4-phenoxyphenyl)-1H-pyrazol[3,4-d]pyrimidin-1-y1]-1-piperidinyl]-2-propen-1-on (Ibrutinib), 4-{8-Amino-3-[(2S)-1-(but-2-ynoyl)pyrrolidin-2-yl]imidazo[1,5-a]pyrazin-1-y1)}-N-(pyridin-2-yl)benzamid (Acalabrutinib), (S)-7-(1-Acryloylpiperidin-4-yl)-2-(4-phenoxyphenyl)-4,5,6,7-tetrahydropyrazol[1,5-a]pyrimidin-3-carboxamid (Zanubrutinib), N-(3-((2-((3-Fluor-4-(4-methylpiperazin-1-yl)phenyl)amino)-7H-pyrrol[2,3-d]pyrimidin-4-yl)oxy)phenyl)acrylamid (Abivertinib), 1-(4-(((6-Amino-5-(4-phenoxyphenyl)pyrimidin-4-yl)amino)methyl)piperidin-1-yl)prop-2-en-1-on (Evobrutinib), 4-Amino-3-(4-phenoxyphenyI)-1-[(3R)-1-prop-2-enoylpiperidin-3-yl]imidazo[4,5-c]pyridin-2-on (Tolebrutinib), (E)-2-[(3R)-3-[4-Amino-3-(2-fluor-4-phenoxyphenyl)pyrazol[3,4-d]pyrimidin-1-yl]piperidin-1-carbonyl]-4-methyl-4-[4-(oxetan-3-yl)piperazin-1-yl]pent-2-ennitril (Rilzabrutinib), (2-Chlor-4-phenoxyphenyl) (4-(((3R,6S)-6-(hydroxymethyl)tetrahydro-2H-pyran-3-yl)amino)-7h-pyrrol(2,3-d)pyrimidin-5-yl)methanon (Nemtabrutinib), 4-[(3S)-3-(But-2-ynoylamino)piperidin-1-y1]-5-fluor-2,3-dimethyl-1H-indol-7-carboxamid (Branebrutinib), (S)-4-(1-Acryloylpiperidin-3-yl)-1H-indol-7-carboxamid (Elsubrutinib), 10-[3-(Hydroxymethyl)-4-[(1-methyl-5-[[5-[(2S)-2-methyl-4-(oxetan-3-yl)piperazin-1-yl]pyridin-2-yl]amino]-6-oxopyridin-3-yl]pyridin-2-yl]-4,4-dimethyl-1,10-diazatricyclo[6.4.0.02,6]dodeca-2(6),7-dien-9-on (Fenebrutinib), 5-Amino-3-[4-[[(5-fluor-2-methoxybenzoyl)amino]methyl]pheny1]-1-[(2S)-1,1,1-trifluorpropan-2-yl]pyrazol-4-carboxamid (Pirtobrutinib), 3-[1-[[3-[5-[(1-Methylpiperidin-4-yl)methoxy]pyrimidin-2-yl]phenyl]methy1]-6-oxopyridazin-3-yl]benzonitril (Tepotinib), 2-(4-Phenoxyphenyl)-6-(1-prop-2-enoylpiperidin-4-yl)pyridin-3-carboxamid (Orelabrutinib), N-[3-[6-Amino-5-[2-[methyl(prop-2-enoyl)amino]ethoxy]pyrimidin-4-y1]-5-fluor-2-methylphenyl]-4-cyclopropyl-2-fluorbenzamid (Remibrutinib), 6-Amino-9-[(3R)-1-but-2-ynoylpyrrolidin-3-y1]-7-(4-phenoxyphenyl)purin-8-on (Tirabrutinib), (3R,4S)-1-(6-Amino-5-fluorpyrimidin-4-yl)-3-[(3R)-3-[3-chlor-5-(trifluormethyl)anilin]-2-oxopiperidin-1-yl]piperidin-4-carboxamid (Vecabrutinib), N-[[2-Methyl-4-[2-[(1-methylpyrazol-4-yl)amino]pyrimidin-4-yl]phenyl]methyl]-3-propan-2-yloxyazetidin-1-carboxamid (BiiB068), 1-(3-Fluor-4-(7-(4-methyl-1H-imidazol-2-yl)-1-oxoisoindolin-4-yl)phenyl)-3-(3-(trifluormethyl)phenyl)harnstoff (CG-806), 4-Amino-1-[(3R)-1-but-2-ynoylpyrrolidin-3-y1]-3-[4-(2,6-difluorphenoxy)pheny1]-6H-pyrrol[2,3-d]pyridazin-7-on (Edralbrutinib), N-(3-((2-((4-(4-Methylpiperazin-1-yl)phenyl)amino)furo[3,2-d]pyrimidin-4-yl)oxy)phenyl)acrylamid (Poseltinib), N-[3-[[5-Fluor-2-[4-(2-methoxyethoxy)anilin]pyrimidin-4-yl]amino]phenyl]prop-2-enamid (Spebrutinib), 2-Cyano-N-(2,5-dibromphenyl)-3-hydroxy-2-butenamid (LFM-A13), (1R,65)-3-Hydroxy-4-methyl-7-oxabicyclo[4.1.0]hept-3-en-2,5-dion((-)-terreinsäure), 1-Cyclopentyl-3-(4-phenoxyphenyl)-1H-pyrazol[3,4-d]pyrimidin-4-amin (PCI 29732), 1-[3-Fluor-4-[7-(5-methyl-1H-imidazol-2-yl)-1-oxo-2,3-dihydroisoindol-4-yl]phenyl]-3-(2,4,6-trifluorphenyl)harnstoff (Luxeptinib), (E)-2-[(3R)-3-[4-Amino-3-(2-fluor-4-phenoxyphenyl)pyrazol[3,4-d]pyrimidin-1-yl]piperidin-1-carbony1]-4,4-dimethylpent-2-ennitril (Atuzabrutinib), 2-(3-(2-Amino-6-(1-(oxetan-3-yl)-1,2,3,6-tetrahydropyridin-4-y1)-7H-pyrrol[2,3-d]pyrimidin-4-y1)-2-(hydroxymethyl)phenyl)-6-cyclopropyl-8-fluorisochinolin-1(2H)-on (Milrebrutinib), 1-tert-Butyl-N-[8-[2-[(1-methylpyrazol-4-yl)amino]pyrimidin-4-yl]-2-(oxetan-3-yl)-1,3,4,5-tetrahydro-2-benzazepin-5-yl]triazol-4-carboxamid (BIIB-091), N-[3-[2-[4-(4-Methylpiperazin-1-yl)anilin]thien[3,2-d]pyrimidin-4-yl]oxyphenyl]prop-2-enamid (Olmutinib), 2-Propen-1-on,1-[4-[[[6-amino-5-(4-phenoxyphenyl)-4-pyrimidinyl]amino]methyl]-4-fluor-1-piperidinyl] (TL-895) und 2-Pyrazincarboxamid, 3-[[4-[1-[[(3S)-1-[2-(2,6-dioxo-3-piperidinyl)-2,3-dihydro-1,3-dioxo-1H-isoindol-5-y1]-3-pyrrolidinyl]methy1]-4-piperidinyl]phenyl]amino]-5-(1-piperidinyl) (NX2127).

2. BTKi zur Verwendung bei einem Verfahren nach Anspruch 1, wobei der BTKi innerhalb einer Zeitspanne, die vom Aussetzen des Subjekts dem Agens, das die allergische Reaktion auslöst, bis 2 Stunden nach dem Aussetzen reicht, verabreicht wird.

3. BTKi zur Verwendung bei einem Verfahren nach Anspruch 1 oder Anspruch 2, wobei der BTKi innerhalb einer Zeitspanne, die vom Aussetzen des Subjekts dem Agens, das die allergische Reaktion auslöst, bis 30 min nach dem Aussetzen reicht, verabreicht wird.

4. BTKi zur Verwendung bei einem Verfahren nach einem der vorhergehenden Ansprüche, wobei der BTKi dem Subjekt, das dessen bedarf, verabreicht wird, bevor oder wenn das Subjekt, das dessen bedarf, ein erstes klinisches Anzeichen oder Symptom einer allergischen Reaktion erfahren hat, die sich als Folge des Aussetzens entwickelt hat.

5. BTKi zur Verwendung bei einem Verfahren nach einem der vorhergehenden Ansprüche, wobei der BTKi die Degranulierung von Mastzellen und/oder Basophilen in dem Subjekt, das deren bedarf, reduziert, welche Degranulierung durch Aussetzen dem Agens ausgelöst wird, das in der Lage ist, die allergische Reaktion auszulösen.

6. BTKi zur Verwendung bei einem Verfahren nach einem der Ansprüche 1-5, wobei das Verfahren zum Behandeln der allergischen Reaktion Verhindern umfasst, dass eine fortlaufende allergische Reaktion zu einer schweren allergischen Reaktion oder Anaprophylaxie fortschreitet.

7. BTKi zur Verwendung bei einem Verfahren nach einem der Ansprüche 1-5, wobei das Verfahren zum Behandeln der allergischen Reaktion die Notfallbehandlung eines Subjekts umfasst, das unbeabsichtigt einem Agens ausgesetzt wird, das die allergische Reaktion verursacht oder auslöst.

8. BTKi zur Verwendung bei einem Verfahren nach einem der Ansprüche 1-5, wobei das Verfahren zum Behandeln der allergischen Reaktion Reduzieren des Bedarfs des Subjekts für die Verabreichung von Ephedrin und/oder einer Zweitlinienbehandlung mit Antihistamin und/oder Corticosteroiden umfasst.

9. BTKi zur Verwendung bei einem Verfahren nach einem der vorhergehenden Ansprüche, wobei das Subjekt unbeabsichtigt dem Agens ausgesetzt wird.

10. BTKi zur Verwendung bei einem Verfahren nach einem der vorhergehenden Ansprüche, wobei der BTKi als Einzeldosis verabreicht wird, wobei wahlweise eine oder mehrere Dosen auf die erste Dosis hin innerhalb von 24 Stunden von der ersten Dosis verabreicht wird/werden.

11. BTKi zur Verwendung bei einem Verfahren nach einem der vorhergehenden Ansprüche, wobei das Agens ein Allergen, bevorzugt ein Pollenallergen, ein Milbenallergen, ein Nahrungsmittelallergen, ein Insektengiftallergen oder ein Medikament ist.

12. BTKi zur Verwendung bei einem Verfahren nach den Ansprüchen 1-10, wobei das Agens, das in der Lage ist, die allergische Reaktion auszulösen, ein Agens ist, das in der Lage ist, die nicht-IgE-vermittelte Aktivierung von Mastzellen und/oder Basophilen auszulösen.

13. BTKi zur Verwendung bei einem Verfahren nach einem der vorhergehenden Ansprüche, wobei der BTKi als Kombinationstherapie mit Ephedrin verabreicht wird.

14. BTKi zur Verwendung bei einem Verfahren nach einem der vorhergehenden Ansprüche, wobei der BTKi als Zweitlinienarzneimittel als Ersatz für i.v.-Antihistamin oder Corticosteroid verabreicht wird oder als mit Antihistamin oder Corticosteroid verbundener Behandlung verabreicht wird.

15. BTKi zur Verwendung bei einem Verfahren nach einem der vorhergehenden Ansprüche, wobei das Verfahren des Behandelns einer allergischen Reaktion ein Verfahren des Behandelns einer verzögerten allergischen Reaktion oder einer kombiniert akuten und verzögerten allergischen Reaktion ist, wobei die verzögerte allergische Reaktion zwischen 2-48 Stunden erfolgt, nachdem das allergische Subjekt dem Agens ausgesetzt worden ist, das die allergische Reaktion auslöst oder verursacht.

## Revendications

1. BTKi destiné à être utilisé dans un méthode de traitement d'une réaction allergique chez un sujet qui en a besoin, dans lequel la méthode comprend l'administration d'une dose thérapeutiquement efficace d'un inhibiteur de la tyrosine kinase de Bruton (BTKi) audit sujet, dans lequel le BTKi est administré dans une période allant de l'exposition du sujet à un agent déclenchant ladite réaction allergique jusqu'à 24 heures après ladite exposition,
dans lequel le BTKi est choisi dans la liste constituée par la 1-[(3R)-3-[4-amino-3-(4-phénoxyphényl)-1H-pyrazolo[3,4-d]pyrimidin-1-yl]-1-pipéridinyl]-2-propén-1-one (Ibrutinib), le 4-{8-amino-3-[(2S)-1-(but-2-ynoyl)pyrrolidin-2-yl]imidazo[1,5-a]pyrazin-1-yl)}-N-(pyridin-2-yl)benzamide (Acalabrutinib), le (S)-7-(1-acryloylpipéridin-4-yl)-2-(4-phénoxyphényl)-4,5,6,7-tétrahydropyrazolo[1,5-a]pyrimidine-3-carboxamide (Zanubrutinib), le N-(3-((2-((3-fluoro-4-(4-méthylpipérazin-1-yl)phényl)amino)-7H-pyrrolo[2,3-d]pyrimidin-4-yl)oxy)phényl)acrylamide (Abivertinib), la 1-(4-(((6-amino-5-(4-phénoxyphényl)pyrimidin-4-yl)amino)méthyl)pipéridin-1-yl)prop-2-én-1-one (Evobrutinib), la 4-amino-3-(4-phénoxyphényl)-1-[(3R)-1-prop-2-énoylpipéridin-3-yl]imidazo[4,5-c]pyridin-2-one (Tolebrutinib), le (E)-2-[(3R)-3-[4-amino-3-(2-fluoro-4-phénoxyphényl)pyrazolo[3,4-d]pyrimidin-1-yl]pipéridine-1-carbonyl]-4-méthyl-4-[4-(oxétan-3-yl)pipérazin-1-yl]pent-2-ènenitrile (Rilzabrutinib), la (2-chloro-4-phénoxyphényl) (4-(((3R,6S)-6-(hydroxyméthyl)tétrahydro-2H-pyran-3-yl)amino)-7h-pyrrolo(2,3-d)pyrimidin-5-yl)méthanone (Nemtabrutinib), le 4-[(3S)-3-(but-2-ynoylamino)pipéridin-1-yl]-5-fluoro-2,3-diméthyl-1H-indole-7-carboxamide (Branebrutinib), le (S)-4-(1-acryloylpipéridin-3-yl)-1H-indole-7-carboxamide (Elsubrutinib), la 10-[3-(hydroxyméthyl)-4-[1-méthyl-5-[[5-[(2S)-2-méthyl-4-(oxétan-3-yl)pipérazin-1-yl]pyridin-2-yl]amino]-6-oxopyridin-3-yl]pyridin-2-yl]-4,4-diméthyl-1,10-diazatricyclo[6.4.0.02,6]dodéca-2(6),7-dién-9-one (Fenebrutinib), le 5-amino-3-[4-[[(5-fluoro-2-méthoxybenzoyl)amino]méthyl]phényl]-1-[(2S)-1,1,1-trifluoropropan-2-yl]pyrazole-4-carboxamide (Pirtobrutinib), le 3-[1-[[3-[5-[(1-méthylpipéridin-4-yl)méthoxy]pyrimidin-2-yl]phényl]méthyl]-6-oxopyridazin-3-yl]benzonitrile (Tepotinib), le 2-(4-phénoxyphényl)-6-(1-prop-2-énoylpipéridin-4-yl)pyridine-3-carboxamide (Orelabrutinib), le N-[3-[6-amino-5-[2-[méthyl(prop-2-énoyl)amino]éthoxy]pyrimidin-4-yl]-5-fluoro-2-méthylphényl]-4-cyclopropyl-2-fluorobenzamide (Remibrutinib), la 6-amino-9-[(3R)-1-but-2-ynoylpyrrolidin-3-yl]-7-(4-phénoxyphényl)purin-8-one (Tirabrutinib), le (3R,4S)-1-(6-amino-5-fluoropyrimidin-4-yl)-3-[(3R)-3-[3-chloro-5-(trifluorométhyl)anilino]-2-oxopipéridin-1-yl]pipéridine-4-carboxamide (Vecabrutinib), le N-[[2-méthyl-4-[2-[(1-méthylpyrazol-4-yl)amino]pyrimidin-4-yl]phényl]méthyl]-3-propan-2-yloxyazétidine-1-carboxamide (BiiB068), la 1-(3-fluoro-4-(7-(4-méthyl-1H-imidazol-2-yl)-1-oxoisoindolin-4-yl)phényl)-3-(3-(trifluorométhyl)phényl)urée (CG-806), la 4-amino-1-[(3R)-1-but-2-ynoylpyrrolidin-3-yl]-3-[4-(2,6-difluorophénoxy)phényl]-6H-pyrrolo[2,3-d]pyridazin-7-one (Edralbrutinib), le N-(3-((2-((4-(4-méthylpipérazin-1-yl)phényl)amino)furo[3,2-d]pyrimidin-4-yl)oxy)phényl)acrylamide (Poseltinib), le N-[3-[[5-fluoro-2-[4-(2-méthoxyéthoxy)anilino]pyrimidin-4-yl]amino]phényl]prop-2-ènamide (Spebrutinib), le 2-cyano-N-(2,5-dibromophényl)-3-hydroxy-2-butènamide (LFM-A13), la (1R,6S)-3-hydroxy-4-méthyl-7-oxabicyclo[4.1.0]hept-3-ène-2,5-dione ((-)-acide terréique), la 1-cyclopentyl-3-(4-phénoxyphényl)-1H-pyrazolo[3,4-d]pyrimidin-4-amine (PCI 29732), la 1-[3-fluoro-4-[7-(5-méthyl-1H-imidazol-2-yl)-1-oxo-2,3-dihydroisoindol-4-yl]phényl]-3-(2,4,6-trifluorophényl)urée (luxeptinib), le (E)-2-[(3R)-3-[4-amino-3-(2-fluoro-4-phénoxyphényl)pyrazolo[3,4-d]pyrimidin-1-yl]pipéridine-1-carbonyl]-4,4-diméthylpent-2-ènenitrile (Atuzabrutinib), la 2-(3-(2-amino-6-(1-(oxétan-3-yl)-1,2,3,6-tétrahydropyridin-4-yl)-7H-pyrrolo[2,3-d]pyrimidin-4-yl)-2-(hydroxyméthyl)phényl)-6-cyclopropyl-8-fluoroisoquinoléin-1(2H)-one (Milrebrutinib), le 1-tert-butyl-N-[8-[2-[(1-méthylpyrazol-4-yl)amino]pyrimidin-4-yl]-2-(oxétan-3-yl)-1,3,4,5-tétrahydro-2-benzazépin-5-yl]triazole-4-carboxamide (BIIB-091), le N-[3-[2-[4-(4-méthylpipérazin-1-yl)anilino]thiéno[3,2-d]pyrimidin-4-yl]oxyphényl]prop-2-énamide (Olmutinib), la 2-propén-1-one, le 1-[4-[[[6-amino-5-(4-phénoxyphényl)-4-pyrimidinyl]amino]méthyl]-4-fluoro-1-pipéridinyle] (TL-895), et le 2-pyrazinecarboxamide, le 3-[[4-[1-[[(3S)-1-[2-(2,6-dioxo-3-pipéridinyl)-2,3-dihydro-1,3-dioxo-1H-isoindol-5-yl]-3-pyrrolidinyl]méthyl]-4-pipéridinyl]phényl]amino]-5-(1-pipéridinyle) (NX-2127).

2. BTKi destiné à être utilisé dans une méthode selon la revendication 1, dans lequel le BTKi est administré dans une période allant de l'exposition du sujet à l'agent déclenchant ladite réaction allergique jusqu'à 2 heures après ladite exposition.

3. BTKi destiné à être utilisé dans une méthode selon la revendication 1 ou la revendication 2, dans lequel le BTKi est administré dans une période allant de l'exposition du sujet à l'agent déclenchant ladite réaction allergique jusqu'à 30 minutes après ladite exposition.

4. BTKi destiné à être utilisé dans une méthode selon l'une quelconque des revendications précédentes, dans lequel le BTKi est administré audit sujet qui en a besoin avant ou dès que le sujet qui en a besoin présente un premier signe ou symptôme clinique d'une réaction allergique développée suite à ladite exposition.

5. BTKi destiné à être utilisé dans une méthode selon l'une quelconque des revendications précédentes, dans lequel ledit BTKi réduit la dégranulation des mastocytes et/ou des basophiles chez ledit sujet qui en a besoin, laquelle dégranulation est déclenchée par exposition audit agent susceptible de déclencher ladite réaction allergique.

6. BTKi destiné à être utilisé dans une méthode selon l'une quelconque des revendications 1 à 5, dans lequel la méthode de traitement de la réaction allergique comprend la prévention de la progression d'une réaction allergique en cours en une réaction allergique grave ou en un choc anaphylactique.

7. BTKi destiné à être utilisé dans une méthode selon l'une quelconque des revendications 1 à 5, dans lequel la méthode de traitement de la réaction allergique comprend le traitement d'urgence d'un sujet qui a été exposé accidentellement à un agent provoquant ou déclenchant ladite réaction allergique.

8. BTKi destiné à être utilisé dans une méthode selon l'une quelconque des revendications 1 à 5, dans lequel la méthode de traitement de la réaction allergique comprend la réduction du besoin des sujets d'administration d'épinéphrine et/ou d'un traitement de deuxième intention avec antihistaminique et/ou corticoïdes.

9. BTKi destiné à être utilisé dans une méthode selon l'une quelconque des revendications précédentes, dans lequel ledit sujet est exposé accidentellement audit agent.

10. BTKi destiné à être utilisé dans une méthode selon l'une quelconque des revendications précédentes, dans lequel le BTKi est administré sous forme de dose unique, éventuellement dans lequel une ou plusieurs doses sont administrées après la première dose dans les 24 heures suivant la première dose.

11. BTKi destiné à être utilisé dans une méthode selon l'une quelconque des revendications précédentes, dans lequel ledit agent est un allergène, de préférence un allergène de pollen, un allergène d'acarien, un allergène alimentaire, un allergène de venin d'insecte ou un médicament.

12. BTKi destiné à être utilisé dans une méthode selon l'une quelconque des revendications 1 à 10, dans lequel l'agent susceptible de déclencher ladite réaction allergique est un agent susceptible de déclencher une activation non médiée par les IgE des mastocytes et/ou des basophiles.

13. BTKi destiné à être utilisé dans une méthode selon l'une quelconque des revendications précédentes, dans lequel le BTKi est administré sous forme de thérapie combinée avec de l'épinéphrine.

14. BTKi destiné à être utilisé dans une méthode selon l'une quelconque des revendications précédentes, dans lequel le BTKi est administré comme médicament de deuxième intention en remplacement d'un antihistaminique ou d'un corticoïde en i.v. ou est administré comme traitement adjuvant avec un antihistaminique ou un corticoïde.

15. BTKi destiné à être utilisé dans une méthode selon l'une quelconque des revendications précédentes, dans lequel ladite méthode de traitement d'une réaction allergique est une méthode de traitement d'une réaction allergique retardée ou d'une réaction allergique combinée aiguë et retardée, dans lequel la réaction allergique retardée survient entre 2 et 48 heures après l'exposition du sujet allergique à l'agent déclenchant ou provoquant la réaction allergique.
